(19) **European Patent Office**

Europäisches Patentamt
European Patent Office
Office européen des brevets

(11) **EP 4 538 377 A1**

(12) **EUROPEAN PATENT APPLICATION**
published in accordance with Art. 153(4) EPC

(43) Date of publication:
**16.04.2025 Bulletin 2025/16**

(21) Application number: **23811901.0**

(22) Date of filing: **26.05.2023**

(51) International Patent Classification (IPC):
*C12N 15/62* (2006.01)      *C07K 14/315* (2006.01)
*C07K 14/52* (2006.01)      *C07K 14/705* (2006.01)
*C07K 16/00* (2006.01)      *C07K 19/00* (2006.01)
*C12N 15/63* (2006.01)      *C12P 21/00* (2006.01)
*G01N 33/50* (2006.01)

(52) Cooperative Patent Classification (CPC):
**C07K 14/315; C07K 14/52; C07K 14/705;
C07K 16/00; C07K 19/00; C12N 15/62;
C12N 15/63; C12P 21/00; G01N 33/50**

(86) International application number:
**PCT/JP2023/019733**

(87) International publication number:
**WO 2023/229029 (30.11.2023 Gazette 2023/48)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **26.05.2022 JP 2022086339**

(71) Applicants:
• **National University Corporation
Yamagata University
Yamagata-shi, Yamagata 990-8560 (JP)**
• **University Public Corporation Osaka
Osaka City 545-0051 (JP)**
• **National University Corporation
Tokyo University Of Agriculture and Technology
Fuchu-shi
Tokyo 183-8538 (JP)**

(72) Inventors:
• **MAKABE, Koki
Yonezawa-shi, Yamagata 992-8510 (JP)**
• **NAKANISHI, Takeshi
Osaka-shi, Osaka 558-8585 (JP)**
• **ASANO, Ryutaro
Fuchu-shi, Tokyo 183-8538 (JP)**

(74) Representative: **Schön, Christoph
Dr. Schön, Neymeyr & Partner mbB
Bavariaring 26
80336 München (DE)**

Remarks:
The complete document including Reference Table(s) and the Sequence Listing(s) can be downloaded from the EPO website

(54) **HETERODIMERIC PROTEIN PRODUCTION METHOD, DIMERIC PROTEIN, MONOMERIC PROTEIN, AND TARGET RESPONSIVE HETERODIMERIC PROTEIN SCREENING METHOD**

(57)      Provided is a method for producing a heterodimeric protein such as a bispecific antibody with which a heterodimeric protein composed of only domains having natural amino acid sequences can also be produced. A production method of the present invention is a method for producing a heterodimeric protein. The production method includes a production step of producing a heterodimeric protein by reacting a dimeric protein having a reaction tag with a modification protein for modifying the dimeric protein. The dimeric protein having a reaction tag includes a first monomeric protein and a second monomeric protein. The first monomeric protein includes a first reaction tag and a first dimer formation domain capable of forming a dimer in this order. The first reaction tag includes a binding tag and a first C intein. The second monomeric protein includes a second reaction tag and a second dimer formation domain capable of forming a dimer together with the first dimer formation domain in this order. The second reaction tag includes a binding partner capable of binding to the binding tag, and a second C intein. The modification protein includes a first modification protein and a second modification protein.

EP 4 538 377 A1

The first modification protein includes a first N intein capable of reacting with the first C intein, and a first addition component to be added to the first monomeric protein. The second modification protein includes a second N intein capable of reacting with the second C intein, and a second addition component to be added to the second monomeric protein. The first addition component and the second addition component are different addition components. The first monomeric protein and the second monomeric protein form a dimer. **In** the production step, the first N intein of the first modification protein reacts with the first C intein of the first monomeric protein, and thus the first addition component of the first modification protein is linked to the first monomeric protein, and the second N intein of the second modification protein reacts with the second C intein of the second monomeric protein, and thus the second addition component of the second modification protein is linked to the second monomeric protein.

Fig. 1:

FIG. 1A

FIG. 1B　　　　FIG. 1C

FIG. 1D

**Description**

TECHNICAL FIELD

The present invention relates to a method for producing a heterodimeric protein, a dimeric protein, a monomeric protein, and a method for screening a target-reactive heterodimeric protein.

BACKGROUND ART

Multispecific antibodies have antigen-binding domains capable of respectively binding to different antigens in one antibody molecule, and those for use in various pharmaceutical applications such as anticancer agents and antihemophilic drugs have been developed by changing target antigens.

However, there is a problem in that the production efficiency of multispecific antibodies is very low and it is difficult to produce multispecific antibodies. For example, bispecific antibodies are composed of two types of heavy chains (H chains) and two types of light chains (L chains). When the bispecific antibodies are produced through expression of two types of H chains and two types of L chains, the number of combinations of two H chains and two L chains in the expressed antibodies is ten, which leads to production of unnecessary nine antibodies in addition to a target bispecific antibody (Patent Literature 1).

Citation List

Patent Literature

Patent Literature 1: WO 2013/065708

SUMMARY OF INVENTION

Technical Problem

Therefore, in the case of bispecific antibodies, a technique in which mutations are introduced into the amino acid sequences of the Fc regions of the antibodies and thus two different heavy chains specifically associate with each other (this technique is called "knob-into-holes") and other techniques have been developed.

However, with these techniques, unnatural amino acid sequences are present in the Fc regions of the antibody, and thus antibodies against these regions may be generated.

Therefore, it is an object of the present invention to provide a method for producing a heterodimeric protein such as a bispecific antibody with which a heterodimeric protein composed of only domains having natural amino acid sequences can also be produced.

Solution to Problem

In order to achieve the object above, a production method of the present invention (also referred to as a "production method" hereinafter) is a method for producing a heterodimeric protein, the method including a production step of producing a heterodimeric protein by reacting a dimeric protein having a reaction tag with a modification protein for modifying the dimeric protein,

the dimeric protein having a reaction tag including a first monomeric protein and a second monomeric protein,
the first monomeric protein including a first reaction tag and a first dimer formation domain capable of forming a dimer in this order,
the first reaction tag including a binding tag and a first C intein,
the second monomeric protein including a second reaction tag and a second dimer formation domain capable of forming a dimer together with the first dimer formation domain in this order,
the second reaction tag including a binding partner capable of binding to the binding tag, and a second C intein,
the modification protein including a first modification protein and a second modification protein,
the first modification protein including a first N intein capable of reacting with the first C intein, and a first addition component to be added to the first monomeric protein,
the second modification protein including a second N intein capable of reacting with the second C intein, and a second addition component to be added to the second monomeric protein,
the first addition component and the second addition component being different addition components, and

the first monomeric protein and the second monomeric protein forming a dimer,
wherein, in the production step,
the first N intein of the first modification protein reacts with the first C intein of the first monomeric protein, and thus the first addition component of the first modification protein is linked to the first monomeric protein, and
the second N intein of the second modification protein reacts with the second C intein of the second monomeric protein, and thus the second addition component of the second modification protein is linked to the second monomeric protein.

A protein of the present invention (also referred to as a "dimeric protein" hereinafter) includes a dimeric protein having a reaction tag,

wherein the dimeric protein having a reaction tag includes a first monomeric protein and a second monomeric protein,
the first monomeric protein includes a first reaction tag and a first dimer formation domain capable of forming a dimer,
the first reaction tag includes a binding tag and a first C intein capable of reacting with a first N intein,
the second monomeric protein includes a second reaction tag and a second dimer formation domain capable of forming a dimer together with the first dimer formation domain in this order,
the second reaction tag includes a binding partner capable of binding to the binding tag, and a second C intein capable of reacting with a second N intein, and
the first monomeric protein and the second monomeric protein form a dimer.

A protein of the present invention (also referred to as a "first monomeric protein" hereinafter) includes a first monomeric protein,

wherein the first monomeric protein includes a first reaction tag and a first dimer formation domain capable of forming a dimer in this order,
the first reaction tag includes a binding tag capable of binding to a binding partner and a first C intein capable of reacting with a first N intein,
the first monomeric protein is capable of forming a dimer together with a second monomeric protein,
the second monomeric protein includes a second reaction tag and a second dimer formation domain capable of forming a dimer together with the first dimer formation domain, and
the second reaction tag includes a binding partner capable of binding to the binding tag, and a second C intein capable of reacting with a second N intein.

A protein of the present invention (also referred to as a "second monomeric protein" hereinafter) includes a second monomeric protein,

wherein the second monomeric protein includes a second reaction tag and a second dimer formation domain capable of forming a dimer in this order,
the second reaction tag includes a binding partner capable of binding to a binding tag and a second C intein capable of reacting with a second N intein,
the second monomeric protein is capable of forming a dimer together with a first monomeric protein,
the first monomeric protein includes a first reaction tag and a first dimer formation domain capable of forming a dimer together with the second dimer formation domain in this order, and
the first reaction tag includes a binding tag capable of binding to the binding partner, and a first C intein capable of reacting with a first N intein.

A nucleic acid of the present invention encodes the dimeric protein of the present invention, the first monomeric protein of the present invention, and/or the second monomeric protein of the present invention.
An expression vector of the present invention includes the nucleic acid of the present invention.
A method for screening a target-reactive heterodimeric protein according to the present invention (also referred to as a "screening method" hereinafter) includes:

a production step of producing a candidate heterodimeric protein by reacting a dimeric protein having a reaction tag with a modification protein for modifying the dimeric protein;
a detection step of detecting a reaction between the candidate heterodimeric protein and a target by bringing the candidate heterodimeric protein into contact with the target; and
a selection step of selecting the candidate heterodimeric protein used when the reaction is detected as a candidate of a substance that reacts with the target,

wherein the production step is conducted using the production method of the present invention above.

Advantageous Effects of Invention

With the present invention, heterodimeric proteins such as a bispecific antibody that are composed of only domains having natural amino acid sequences can also be produced.

Brief Description of Drawings

[FIG. 1] FIG. 1 shows schematic views illustrating constituent elements of a dimeric protein to be used in a production method of the present invention and examples of steps conducted to produce a heterodimeric protein.
[FIG. 2] FIG. 2 is a photograph showing proteins before and after a PTS reaction and antibody fragments in Example 1.
[FIG. 3] FIG. 3 shows photographs illustrating proteins after a PTS reaction and column purification in Example 1.
[FIG. 4] FIG. 4 is a graph illustrating a protein elution pattern of gel filtration chromatography in Example 1.
[FIG. 5] FIG. 5 is a graph illustrating a binding of a bispecific antibody to CD3-positive cells measured using flow cytometry in Example 1.
[FIG. 6] FIG. 6 is a graph illustrating a binding of a bispecific antibody to HER2-positive cells measured using flow cytometry in Example 1.
[FIG. 7] FIG. 7 shows a graph illustrating a maintained binding of a bispecific antibody to HER2 measured using a surface plasmon resonance method in Example 1.

DESCRIPTION OF EMBODIMENTS

<Definitions>

In this specification, the term "protein" means a peptide polymer composed of unmodified amino acids (natural amino acids), modified amino acids, and/or artificial amino acids. The polymer may be, for example, in the linear form, the branched form, the cyclic form, or the like. The protein can also be referred to as a "peptide" or "polypeptide".
In this specification, the term "monomeric protein" means a protein in a state of not binding to another protein or not associating with another protein.
In this specification, the term "dimeric protein" means a protein complex formed by two proteins or protein subunits binding to or associating with each other. When the two proteins are the same protein or subunit, the dimeric protein can also be referred to as a "homodimeric protein". When the two proteins are different proteins or subunits, the dimeric protein can also be referred to as a "heterodimeric protein".
In this specification, the term "fusion protein" means a protein formed by portions or entireties of two or more different proteins or different types of proteins binding (linked) to each other via a peptide bond. The fusion protein may be a natural fusion protein or an artificial fusion protein. The artificial fusion protein may be, for example, a protein designed using a genetic engineering technique.
In this specification, the term "binding tag" means a polypeptide or substance capable of specifically binding to another molecule.
In this specification, the term "binding partner" means a polypeptide or substance capable of specifically binding to the binding tag.
In this specification, the term "domain" means a region in a "protein", "polypeptide", and/or "peptide" considered as one unit in terms of its three-dimensional structure or function.
In this specification, the term "antibody" means a protein that includes one or more polypeptides each substantially or partially encoded by an immunoglobulin gene or immunoglobulin gene fragment. The immunoglobulin gene includes, for example, a gene that encodes a constant region such as κ, λ, α (including α1 and α2), γ (including γ1, γ2, γ3, and γ4), δ, ε, or μ, and a gene that can encode innumerable immunoglobulin variable regions such as the V region, the D region, and the J region. The antibody includes, for example, a heavy chain and a light chain. The light chain includes κ and λ, which form a κ chain and a λ chain, respectively. The heavy chain includes γ, μ, α, δ, or ε, which form IgG, IgM, IgA, IgD, and IgE of immunoglobulin classes, respectively. The antibody may be a structural unit of a typical immunoglobulin (antibody) composed of a tetramer. In this case, the antibody is composed of two identical polypeptide chain pairs, and each pair is composed of one light chain (about 25 kDa) and one heavy chain (about 50 to 70 kDa). In addition, the N terminus of each chain defines a variable region that is mainly involved in antigen recognition and that is composed of about 100 to 110 or more amino acids.
In this specification, the term "antigen-binding fragment" means a polypeptide that is a part or portion of an antibody and that includes an antigen-binding site. The antigen-binding fragment can be obtained by chemically or enzymatically processing an antibody. The antigen-binding fragment can also be obtained through a recombination technique. Examples of the antigen-binding fragment include Fab, Fab', F(ab')$_2$, Fc, and/or an Fv fragment, and derivatives thereof.

In this specification, the term "heavy chain antibody" means an antibody composed of only two heavy chains. The heavy chain antibody can also be referred to as an "antibody having no light chains".

In this specification, the term "multispecific antibody" means an antibody or antibody derivative that is specific to two or more antigens and/or two or more epitopes.

In this specification, the term "intein" means an autocatalytic enzyme having both protein protease activity and protein ligase activity. For example, in a certain protein, the intein is a protein that cleaves out its own amino acid sequence from the protein through protein splicing and links the residual amino acid sequences (exteins) via a peptide bond. The "intein" can also be referred to as, for example, a "protein intron".

In this specification, the term "split-type intein" means an intein that is composed of two complementary half inteins, namely a C intein and an N intein, the C intein and the N intein intimately binding to each other to form an active intein and exhibiting enzymatic activity. The split-type intein is also referred to as a "split intein".

In this specification, the term "integrated intein" means an intein that is one intein composed of a C intein and an N intein, the C intein and the N intein intimately binding to each other to form an active intein and exhibiting enzymatic activity.

In this specification, the term "complementary inteins" means a combination (pair) of the C intein and the N intein of an intein or split-type intein.

In this specification, the term "C intein" means an intein polypeptide having a homology to the C-terminal moiety of an intein. The C intein binds to a complementary N intein to form an active intein.

In this specification, the term "N intein" means an intein polypeptide having a homology to the N-terminal moiety of an intein. The N intein binds to a complementary C intein to form an active intein.

In this specification, the term "salt" means an electrolyte capable of ionizing in an aqueous solvent. The term "aqueous solvent" means a water-containing solvent.

In this specification, the term "solubilization domain" means a polypeptide that, when fused to a peptide, polypeptide, or protein, increases (e.g., enhances or promotes) the expression level of the peptide, polypeptide, or protein expressed in a transformant compared with expression of the peptide, polypeptide, or protein without the solubilization domain.

In this specification, the term "purification" means identification and separation of a substance, collection of a substance from components in a natural state, a state in which a substance is identified and separated, and/or a state in which a substance is collected from components in a natural state. The "purification" can be achieved by, for example, obtaining at least one purification step. The purification can also be referred to as "isolation".

In this specification, the term "separation" means separation of a target substance from a substance containing the target substance, and/or a state in which a target substance is separated from a substance containing the target substance. The separation can also be referred to as "liberation".

In this specification, the term "nucleic acid" means a polymer of deoxyribonucleotides (DNAs), ribonucleotides (RNAs), and/or altered nucleotides. In this specification, when the "nucleic acid" is used in combination with a specific protein, the "nucleic acid" means a polymer of nucleotides encoding the amino acid sequence of the protein. Examples of the nucleic acid include genome DNA, cDNA, mRNA, and the like. The nucleic acid may be, for example, a single-stranded nucleic acid, a double-stranded nucleic acid, or the like. The nucleic acid is interchangeable with "polynucleotide" or "nucleic acid molecule".

In this specification, the term "host" means a cell and/or an individual into which an exogenous nucleic acid is to be introduced. When the host is a cell, the host can also be referred to as a "host cell".

In this specification, the terms "vector" and "expression vector" mean a recombinant plasmid or virus that includes a nucleic acid to be delivered to a host or host cell in vitro or in vivo. Examples of the "vector" and the "expression vector" include viral vectors and non-viral vectors.

In this specification, the term "transformant" means a host into which an exogenous nucleic acid has been introduced.

In this specification, the origin of the protein, polypeptide, or peptide is not particularly limited and is any animal. The animal is, for example, a human or a non-human animal. Examples of the non-human animal include mammals such as a mouse, a rat, a rabbit, a dog, a cat, a cow, a horse, a pig, a monkey, a dolphin, and a sea lion.

Although the following describes examples of the present invention, the present invention is not limited to the following examples and the like, and can be implemented with any modifications. Also, each description in the present invention can be applied to other descriptions unless otherwise stated. Note that the expression "to" as used herein means that the numerical or physical values before and after "to" are included. In addition, the expression "A and/or B" as used herein encompasses "only A", "only B", and "both A and B".

<Method for Producing Heterodimeric Protein>

An aspect of the present invention provides a method for producing a heterodimeric protein such as a bispecific antibody with which a heterodimeric protein composed of only domains having natural amino acid sequences can also be produced, or a method with which heterodimeric proteins modified with different addition components can be produced. The production method of the present invention is a method for producing a heterodimeric protein, the method including a production step of producing a heterodimeric protein by reacting a dimeric protein having a reaction tag with a modification protein for modifying the dimeric protein, the dimeric protein having a reaction tag including a first monomeric protein and a second monomeric protein, the first monomeric protein including a first reaction tag and a first dimer formation domain capable of forming a dimer in this order, the first reaction tag including a binding tag and a first C intein, the second monomeric protein including a second reaction tag and a second dimer formation domain capable of forming a dimer together with the first dimer formation domain in this order, the second reaction tag including a binding partner capable of binding to the binding tag, and a second C intein, the modification protein including a first modification protein and a second modification protein, the first modification protein including a first N intein capable of reacting with the first C intein, and a first addition component to be added to the first monomeric protein, the second modification protein including a second N intein capable of reacting with the second C intein, and a second addition component to be added to the second monomeric protein, the first addition component and the second addition component being different addition components, and the first monomeric protein and the second monomeric protein forming a dimer, wherein, in the production step, the first N intein of the first modification protein reacts with the first C intein of the first monomeric protein, and thus the first addition component of the first modification protein is linked to the first monomeric protein, and the second N intein of the second modification protein reacts with the second C intein of the second monomeric protein, and thus the second addition component of the second modification protein is linked to the second monomeric protein.

When the first monomeric protein and the second monomeric protein are proteins that form a homodimer, dimers composed of various combinations are produced by preparing the first monomeric protein linked to the first addition component and the second monomeric protein linked to the second addition component in advance and binding these proteins. Meanwhile, in the production method of the present invention, the first addition component is linked to the first monomeric protein by utilizing a reaction between the first N intein and the first C intein in the state in which the dimeric protein is formed. In addition, in the production method of the present invention, the second addition component is linked to the second monomeric protein by utilizing a reaction between the second N intein and the second C intein in the state in which the dimeric protein is formed. Accordingly, with the production method of the present invention, it is possible to efficiently produce a heterodimeric protein composed of a protein derived from the first monomeric protein linked to the first addition component and a protein derived from the second monomeric protein linked to the second addition component even when the first monomeric protein and the second monomeric protein are proteins that form a homodimer.

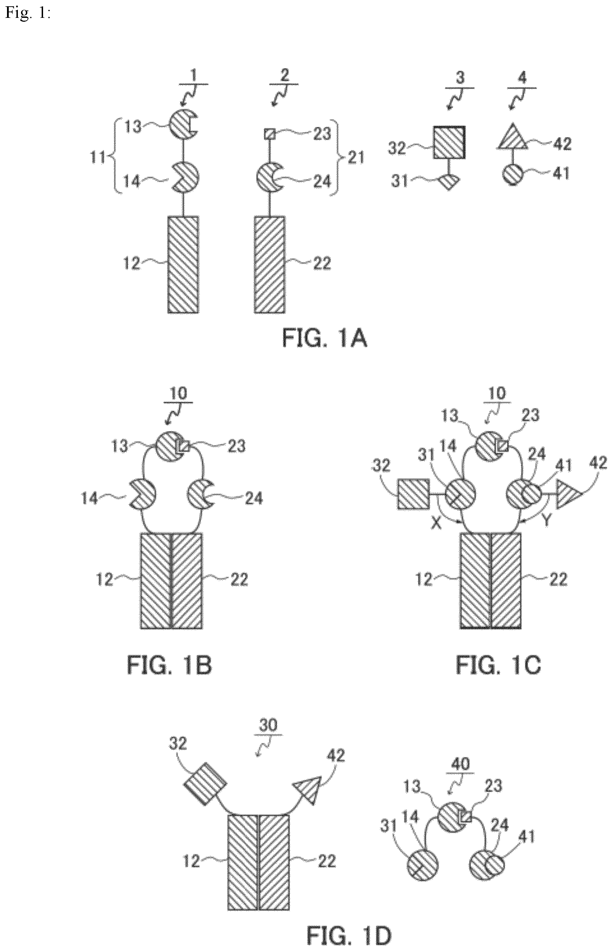

The inventors of the present invention came up with an idea that different addition components are linked to two monomeric proteins through binding reactions specific to the monomeric proteins in the state in which the monomeric proteins form a dimer, thus making it possible to produce a heterodimeric protein composed of the monomeric proteins linked to the different addition components. Then, as a result of extensive research, the inventors of the present invention found that the idea above could be implemented by using two types of monomeric proteins to each of which a binding tag or binding partner and one of different inteins have been added and two types of modification proteins to each of which one of inteins capable of reacting with the different inteins and the addition component for the monomeric protein are added, and thus achieved the present invention. Specifically, it is assumed that, with the production method of the present invention, a heterodimeric protein composed of monomeric proteins linked to different addition components can be produced in accordance with a mechanism described below as shown in FIG. 1. However, the present invention is not limited to the mechanism described below.

As shown in FIG. 1(A), with the production method of the present invention, a heterodimeric protein can be produced using a first monomeric protein 1, a second monomeric protein 2, a first modification protein 3, and a second modification protein 4. The first monomeric protein 1 includes a first reaction tag 11 and a first dimer formation domain 12 in this order from the N terminus side. The first reaction tag 11 includes a binding tag 13 and a first C intein 14 in this order from the N terminus side. The second monomeric protein 2 includes a second reaction tag 21 and a second dimer formation domain 22 in this order from the N terminus side. The second reaction tag 21 includes a binding partner 23 and a second C intein 24 in this order from the N terminus side. The first modification protein 3 includes a first N intein 31 and a first addition component 32 in this order from the N terminus side. The second modification protein 4 includes a second N intein 41 and a second addition component 42 in this order from the N terminus side.

First, a dimeric protein 10 that is a dimer formed by the first monomeric protein 1 and the second monomeric protein 2 is prepared as shown in FIG. 1(B) by bringing the first monomeric protein 1 and the second monomeric protein 2 into contact with each other. Next, the dimeric protein 10 is brought into contact with the first modification protein 3 and the second modification protein 4, and thus, as shown in FIG. 1(C), the first N intein 31 of the first modification protein 3 and the first C intein 14 of the first monomeric protein 1 form an active intein and cause a reaction (arrow X). Also, the second N intein 41 of the second modification protein 4 and the second C intein 24 of the second monomeric protein 2 form an active intein and cause a reaction (arrow Y). Then, the peptide bond between the first C intein 14 and the first dimer formation domain 12 and the peptide bond between the first addition component 32 and the first N intein 31 are rearranged, and the first dimer formation domain 12 is linked to the C terminus side of the first addition component 32. Also, the peptide bond between the second C intein 24 and the second dimer formation domain 22 and the peptide bond between the second addition component 42 and the second N intein 41 are rearranged, and the second dimer formation domain 22 is linked to the C terminus side of the second addition component 42. As a result, as shown in FIG. 1(D), a heterodimeric protein 30 can be produced in which the first dimer formation domain 12 and the second dimer formation domain 22, which form the dimer, are modified with the different addition components 32 and 42. Therefore, it is assumed that, with the production method of the present invention, a heterodimeric protein modified with a desired addition component can be produced.

In the production method of the present invention, the first addition component 32 and the second addition component 42 are added using inteins. Accordingly, the monomeric proteins to be included in the obtained heterodimeric protein can form the heterodimeric protein while being in the form of a fusion protein in which portions are linked together via a peptide bond from the N terminus to the C terminus similarly to recombinant proteins. Therefore, with the production method of the present invention, a heterodimeric protein in which a plurality of domains having only a natural amino acid sequence are linked together can also be produced by using the first dimer formation domain 12, the second dimer formation domain 22, the first addition component 32, and the second addition component 42 having a natural amino acid sequence, that is, an amino acid sequence that includes no amino acid mutation or no artificial amino acid sequence. Therefore, for example, it is possible to favorably produce a bispecific monoclonal antibody having a natural amino acid sequence by using, as the first addition component 32 and the second addition component 42, Fabs that bind to different antigens and using, as the first dimer formation domain 12 and the second dimer formation domain 22, proteins composed of the hinge region and the CH2 and CH3 regions in the Fc region of an antibody. As described above, with the production method of the present invention, even when, for example, a heterodimeric protein is produced using two types of monomeric proteins that have the dimer formation domains having a common amino acid sequence and the remainders having different amino acid sequences, it is possible to specifically produce the heterodimeric protein without introducing, into the amino acid sequence, such a mutation that causes specific binding or association between the target monomeric proteins.

Although the N terminal portions of the first dimer formation domain 12 and the second dimer formation domain 22 are modified with the first addition component 32 and the second addition component 42 in the examples illustrated in FIGS. 1(A) to 1(D), the present invention is not limited thereto, and the C terminal portions of the first dimer formation domain 12 and the second dimer formation domain 22 may be modified with the first addition component 32 and the second addition component 42. In this case, the first monomeric protein 1 includes the first reaction tag 11 and the first dimer formation domain 12 in this order from the C terminus side, and the first reaction tag 11 includes the binding tag 13 and the first N intein 31 in this order from the C terminus side. The second monomeric protein 2 includes the second reaction tag 21 and the second dimer formation domain 22 in this order from the C terminus side, and the second reaction tag 21 includes the binding partner 23 and the second N intein 41 in this order from the C terminus side. The first modification protein 3 includes the first C intein 14 and the first addition component 32 in this order from the C terminus side. The second modification protein 4 includes the second C intein 24 and the second addition component 42 in this order from the C terminus side. Except these points, in the production method of the present invention, the C terminal portions of the first dimer formation domain 12 and the second dimer formation domain 22 can be modified with the first addition component 32 and the second addition component 42, and in the following descriptions, the description of the production method can be referred to with the domains being interchanged as described above.

In the production method of the present invention, the dimeric protein having the reaction tags is formed by dimerizing the first monomeric protein and the second monomeric protein. The first monomeric protein and the second monomeric protein can be prepared using, for example, a genetic engineering technique as described below in the description of a method for producing a monomeric protein according to the present invention. Accordingly, the production method of the present invention may optionally include a formation step of forming a dimer through a reaction between the first monomeric protein and the second monomeric protein prior to the production step. Also, the production method of the present invention may optionally include a preparation step of preparing the first monomeric protein and/or the second monomeric protein, and a formation step of forming a dimer through a reaction between the first monomeric protein and the second monomeric protein, prior to the production step. Regarding a preparation method in the preparation step, the descriptions below about the first monomeric protein, the second monomeric protein, the nucleic acid, the expression vector, the transformant, and the method for producing a monomeric protein, which will be described below, can be referred to.

In the formation step, the first monomeric protein and the second monomeric protein are reacted. As a result, as shown in FIG. 1(B), a dimeric protein having the reaction tags can be prepared or provided in the formation step due to the first monomeric protein and the second monomeric protein forming a dimer. The first monomeric protein and the second monomeric protein can form a heterodimer via the binding tag and the binding partner. Accordingly, the formation step may be conducted in parallel with the production step, which will be described below.

It is preferable to cause the reaction in the formation step in the presence of, for example, an aqueous solvent such as a buffer solution, physiological saline, or water. The first monomeric protein and the second monomeric protein may be in the form of a solid or the form of a liquid, but the liquid form is preferable. When the first monomeric protein and the second monomeric protein are in the form of a solid such as powder, it is preferable that the first monomeric protein and the second monomeric protein are dispersed in an aqueous solvent such as a buffer solution, physiological saline, or water in advance. When the first monomeric protein and the second monomeric protein are in the form of a liquid, the first monomeric protein and the second monomeric protein may be diluted using an aqueous solvent such as a buffer solution, physiological saline, or water in advance. Specifically, for example, the reaction can be caused by mixing a solution containing the first monomeric protein and a solution containing the second monomeric protein.

When the first monomeric protein and the second monomeric protein obtained in the preparation step are used as the first monomeric protein and the second monomeric protein, the first monomeric protein and the second monomeric protein may be purified or crude proteins, or a transformant expressing the first monomeric protein and/or the second monomeric protein or a processed product obtained by processing the transformant. The processed product may be, for example, a lysate, an extract, a homogenate, a protein preparation, or a crude product or a purified product thereof, etc. The processed product may be, for example, a product that has been purified at least once.

The first monomeric protein includes, for example, the first reaction tag and the first dimer formation domain capable of forming a dimer in this order from the N terminus side.
The first reaction tag includes, for example, the binding tag and the first C intein in this order from the N terminus side. The second monomeric protein includes, for example, the second reaction tag and the second dimer formation domain capable of forming a dimer together with the first dimer formation domain in this order from the N terminus side. The second reaction tag includes the binding partner and the second C intein. Placing the first reaction tag on the N terminus side of the first dimer formation domain in the first monomeric protein makes it possible to introduce the first addition component to the N terminus side of the first dimer formation domain when the first C intein and the first N intein react. Placing the second reaction tag on the N terminus side of the second dimer formation domain in the second monomeric protein makes it possible to introduce the second addition component to the N terminus side of the second dimer formation domain when the second C intein and the second N intein react.

In the formation step, the first monomeric protein and the second monomeric protein may form a dimer through, for example, (1) a binding between the first reaction tag and the second reaction tag (e.g., a binding between the binding tag of the first reaction tag and the binding partner of the second reaction tag), (2) a binding between the first dimer formation domain and the second dimer formation domain, or bindings of both (1) and (2), but it is preferable that the dimer is formed through the bindings of both (1) and (2) because the dimer formation ability can be improved. It can also be said that the reaction tags are, for example, domains in the first monomeric protein and the second monomeric protein that contribute to the formation of a binding between the monomeric protein and the modification protein and to the addition reaction of adding the addition component to the monomeric protein. In addition, it can also be said that the reaction tags are, for example, domains that are lost from the monomeric proteins after the addition reaction.

The dimer may be formed through a direct binding between the first monomeric protein and the second monomeric protein, an indirect binding (association) between the first monomeric protein and the second monomeric protein, or both the direct binding and the indirect binding between the first monomeric protein and the second monomeric protein. The direct binding is a covalent bond, and specific examples thereof include an amide bond (peptide bond) between amino acids, a disulfide bond between cysteines, and the like. The indirect binding is a noncovalent bond, and specific examples thereof include a hydrogen bond, a hydrophobic bond, and the like.

The first dimer formation domain and the second dimer formation domain can have amino acid sequences capable of forming a dimer in a condition-dependent manner or in a condition-independent manner when proteins that include the domains coexist. Specifically, for example, the amino acid sequences of subunits of a protein dimer or motif sequences included in the dimer can be used as the amino acid sequences of the first dimer formation domain and the second dimer formation domain. The amino acid sequences of subunits of a protein multimer or motif sequences included in the dimer may be used as the amino acid sequences of the first dimer formation domain and the second dimer formation domain. The protein dimer may be a homodimer or a heterodimer. Examples of the protein dimer include immunoglobulins (antibodies) such as IgA, IgD, IgE, IgG, and IgM; proteins with a leucine zipper such as myc family proteins (e.g., AP-1 (c-fos and c-jun), myc, max, mdx1, and the like); G protein-coupled receptors; kinesins; receptor-type tyrosine kinases such as the ErbB receptor family (e.g., an epidermal growth factor receptor (EGFR)), a platelet-derived growth factor receptor (PDGFR), a neurotrophin (neurotrophic factor) receptor, an insulin receptor, an insulin-like growth factor receptor, a vascular endothelial growth factor receptor (VEGFR), and a stem cell factor receptor; Toll-like receptors such as TLR1 to TLR11; and the like.

When the dimer formation motif sequences of an antibody are used as the first dimer formation domain and the second dimer formation domain, the first dimer formation domain and the second dimer formation domain include, for example, amino acid sequences that include the CH2 and CH3 regions in the Fc region of the antibody, or the CH2 and CH3 regions in the Fc region and the hinge region of the antibody. When the first dimer formation domain and the second dimer formation domain include the Fc region of the antibody, the first dimer formation domain and the second dimer formation domain include all or a portion of the amino acid sequence of the Fc region of the antibody. When the first dimer formation domain and the second dimer formation domain include the CH2 and CH3 regions in the Fc region and the hinge region of the antibody, the first dimer formation domain and the second dimer formation domain include all or portions of the amino acid sequences of the CH2 and CH3 regions in the Fc region of the antibody and all or a portion of the amino acid sequence of the hinge region of the antibody, and preferably all the amino acid sequences of the CH2 and CH3 regions in the Fc region of the antibody and all or a portion of the amino acid sequence of the hinge region of the antibody. When the first dimer formation domain and the second dimer formation domain include the CH2 and CH3 regions in the Fc region and the hinge region of the antibody, the first dimer formation domain and the second dimer formation domain include, for example, continuous amino acid sequences in the amino acid sequences of the CH2 and CH3 regions in the Fc region and the hinge region of the antibody. The amino acid sequence of the Fc region or the amino acid sequences of the CH2 and CH3 regions in the Fc region and the hinge region may be natural amino acid sequences (i.e., amino acid sequences with no mutations), or unnatural amino acid sequences (i.e., amino acid sequences with a mutant or population amino acid sequence).

The antibody is, for example, IgA, IgD, IgE, IgG, or IgM, among which IgG is preferable. The IgG is, for example, IgG1, IgG2, IgG2a, IgG2b, IgG3, or IgG4. The antibody is, for example, an animal-derived antibody, and specific examples thereof include a human antibody, a mouse antibody, an avian antibody, a rat antibody, a rabbit antibody, and the like. The first dimer formation domain and the second dimer formation domain are preferably human-derived antibodies and more preferably human-derived IgGs.

Regarding the amino acid sequences of the human IgG1, IgG2, IgG3, and IgG4, for example, the amino acid sequences registered as UniProt Accession Nos. P01857, P01859, P01860, and P01861 can be referred to, respectively.

When the dimer formation motif sequences of an antibody are used as the first dimer formation domain and the second dimer formation domain, the antibody may be an Fc region-altered antibody. In this case, the first dimer formation domain and the second dimer formation domain may include an amino acid sequence that includes the Fc region of the Fc region-altered antibody, or the Fc region and hinge region of the Fc region-altered antibody. Examples of the Fc region-altered antibody include Fcab (Fc antigen binding, References 1 and 5) to which an ability to bind a target molecule is imparted by altering the amino acid sequence of the Fc region, an IgG hexamer (References 2 to 4) to which an ability to form a hexamer is imparted by altering the amino acid sequence of the Fc region of an IgG antibody, DAF (Dual Action Fab, Reference 5), Charge pair (Amgen Inc., Reference 5), SEEDbody (Reference 5), Knobs-in-holes (Reference 5), DVI-IgG (Reference 5), and the like.

Reference 1: G. Wozniak-Knopp et al., "Introducing antigen-binding sites in structural loops of immunoglobulin constant domains: Fc fragments with engineered HER2/neu-binding sites and antibody properties", Protein Engineering, Design and Selection, Volume 23, Issue 4, April 2010, Pages 289-297

Reference 2: Sopp, J.M. et al., "On-target IgG hexamerisation driven by a C-terminal IgM tail-piece fusion variant confers augmented complement activation. ", Commun. Biol., 4, 1031 (2021).

Reference 3: de Jong RN et al., "A Novel Platform for the Potentiation of Therapeutic Antibodies Based on Antigen-Dependent Formation of IgG Hexamers at the Cell Surface. ", PLoS. Biol. (2016) 14(1): e1002344.

Reference 4: Christoph A. Diebolder et al., "Complement Is Activated by IgG Hexamers Assembled at the Cell Surface", Science, 343 (6176), pages 1260-1263

Reference 5: Christoph Spiess et al., "Alternative molecular formats and therapeutic applications for bispecific antibodies", Molecular Immunology, Volume 67, Issue 2, Part A, 2015, Pages 95-106

When the dimer formation motif sequences of an antibody are used as the first dimer formation domain and the second dimer formation domain, the antibody may be a multispecific antibody. In this case, the first dimer formation domain and the second dimer formation domain may include an amino acid sequence that includes the Fc region of the multispecific antibody, or the CH2 and CH3 regions in the Fc region and the hinge region, and a domain added to the C terminus of the Fc region. Examples of the multispecific antibody include IgG-scFv (Reference 5), IgG(L,H)-scFv (Reference 5), IgG(H)-V (Reference 5), KIH IgG-scFab (Reference 5), IgG-2scFv (Reference 5), Intrabody (Reference 5), Tetravalent HCAb (Reference 5), and the like.

The binding tag and the binding partner are such molecules that the binding tag and the binding partner bind together in a condition-dependent manner or in a condition-independent manner when a protein that includes the binding tag and a protein that includes the binding partner coexist. The binding between the binding tag and the binding partner may be a direct binding or an indirect binding.

When the binding between the binding tag and the binding partner is a direct binding, for example, a peptide tag and a peptide that can spontaneously form a covalent bond or a peptide tag and a peptide that can form a covalent bond due to modification activity of another molecule can be used as the binding tag and the binding partner.

When the peptide tag and the peptide that can spontaneously form a covalent bond are used, examples of the binding tag and the binding partner include a Streptococcus pyogenes surface protein (SpyCatcher, SEQ ID NO: 1) and a peptide tag (SpyTag, SEQ ID NO: 2) capable of binding to the SpyCatcher, altered products thereof, and the like. Examples of the altered products of the SpyCatcher and the SpyTag include SpyCatcher2 and SpyTag2 (Reference 6), SpyCatcher3 and SpyTag3 (Reference 7), SnoopCatcher and SnoopTag (Reference 8), and the like.

Reference 6: Anthony H. Keeble et al., "Evolving Accelerated Amidation by SpyTag/SpyCatcher to Analyze Membrane Dynamics", Angew. Chem. Int. Ed., 2017, 56, pages 16521 - 16525

Reference 7: Anthony H. Keeble et al., "Approaching infinite affinity through engineering of Peptide-protein interaction", PNAS, 2019, vol. 116, No. 52, pages 26523-26533

Reference 8: Veggiani G, Nakamura T, Brenner MD, Gayet RV, Yan J, Robinson CV, Howarth M. Programmable polyproteams built using twin peptide superglues. Proc Natl Acad Sci U S A. 2016 Feb 2;113(5):1202-7. doi: 10.1073/pnas.1519214113.

Amino acid sequence of Streptococcus pyogenes surface protein (SpyCatcher) (SEQ ID NO: 1)

DSATHIKFSKRDEDGKELAGATMELRDSSGKTISTWISDGQVKDFYLYPGKYTF

VETAAPDGYEVATAITFTVNEQGQVTVN

Amino acid sequence of SpyTag (SEQ ID NO: 2)

AHIVMVDAYKPTK

When the peptide tag and the peptide that can form a covalent bond due to modification activity of the other molecule are used, examples of the binding tag and the binding partner include a K tag and a Q tag, and the like. A covalent bond can be formed between the K tag and the Q tag by, for example, forming a cross-link between the lysine residue at the N terminus of the K tag and the glutamic acid at the N terminus of the Q tag using bacterial transglutaminase.

When the binding between the binding tag and the binding partner is an indirect binding, for example, an affinity tag and a molecule binding to the affinity tag can be used as the binding tag and the binding partner. The binding tag and the binding partner are, for example, peptides, polypeptides, or proteins. Examples of the binding tag include a His-tag (His×6), a His-Strep-tag, a strep-tag, an avidin tag, a flag (trademark)-tag, an HA (hemagglutinin)-tag, a T7-tag, a V5-peptide-tag, a GST (glutathione-S-transferase)-tag, a CBP (calmodulin-binding peptide)-tag, an MBP (maltose-binding protein)-tag, a Myc-tag, and the like. When the binding partner is a molecule that specifically binds to a target molecule such as an antibody or an antigen-binding fragment thereof, or a derivative thereof, the binding tag may be a peptide having any amino acid sequence to which the molecule that specifically binds to a target molecule can bind.

The binding partner can be selected as appropriate in accordance with the type of the binding tag. Specific examples of the binding partner include an antibody that recognizes the binding tag or an antigen-binding fragment thereof, or a derivative thereof; a nucleic acid molecule such as an aptamer; glutathione, calmodulin; a sugar chain such as mannose; a metal such as nickel, cobalt, or zinc or an ion thereof; and the like.

The combination of the binding tag and the binding partner need only be a combination that enables binding between the binding tag and the binding partner. Specifically, for example, when the binding tag includes a His-tag, the binding partner is, for example, nickel. When the binding tag includes a strep-tag or an avidin tag, the binding partner includes, for example, biotin. When the binding tag includes an epitope tag such as a flag (trademark)-tag, an HA-tag, a T7-tag, a V5-peptide-tag, and/or a Myc-tag, examples of the binding partner include an antibody against the epitope tag or an antigen-binding fragment thereof, or a derivative thereof, or the like. When the binding tag includes a GST-tag, the binding partner is, for example, glutathione. When the binding tag includes a CBP-tag, the binding partner is, for example, calmodulin. When the binding tag includes an MBP-tag, the binding partner is, for example, mannose.

Functional equivalents may be used as the binding tag and the binding partner as long as an ability to establish a binding between the binding tag and the binding partner is maintained. When the binding tag and the binding partner are peptides, polypeptides, or proteins, the functional equivalents may be, for example, polypeptides that have an amino acid sequence having 70% or more, 75% or more, 80% or more, 85% or more, 90% or more, 95% or more, 96% or more, 97% or more, 98% or more, or 99% or more identity to the reference amino acid sequence of the binding tag or the binding partner and that have an ability to bind to the corresponding binding tag or binding partner. The functional equivalents may be, for example, polypeptides that have amino acid sequences consisting of the reference amino acid sequences of the binding tag and the binding partner with deletion, substitution, insertion, and/or addition of one or several amino acids and that have an ability to bind to the corresponding binding tag or the binding partner. The "one or several" amino acids above refer to, for example, 1 to 44 amino acids, 1 to 33 amino acids, 1 to 22 amino acids, 1 to 11 amino acids, 1 to 8 amino acids, 1 to 6 amino acids, 1 to 4 amino acids, 1 to 3 amino acids, 1 or 2 amino acids, or 1 amino acid. The substitution is preferably, for example, conservative substitution.

The first monomeric protein may have one binding tag or a plurality of binding tags. In the latter case, one type of binding tag or a plurality of types of binding tags may be used. When the N terminus of the first monomeric protein is modified with the first addition component (i.e., the first addition component is added to the N terminus of the first monomeric protein), it is preferable to place the binding tag on the N terminus side of the first dimer formation domain.

The second monomeric protein may have one binding partner or a plurality of binding partners. In the latter case, one type of binding partner or a plurality of types of binding partners may be used. When the N terminus of the second monomeric protein is modified with the second addition component (i.e., the second addition component is added to the N terminus of the second monomeric protein), it is preferable to place the binding partner on the N terminus side of the second dimer formation domain. The numbers of the binding partners and the binding partners may be the same or different, but are preferably the same.

In the first monomeric protein and the second monomeric protein, the binding tag and the binding partner are interchangeable, and those included in the combination in the description above may be interchanged and used.

The first C intein and the first N intein are such molecules that the binding tag and the binding partner bind together in a condition-dependent manner or in a condition-independent manner when the first monomeric protein that includes the first C intein and the first modification protein that includes the first N intein coexist. The second C intein and the second N intein are such molecules that the binding tag and the binding partner bind together in a condition-dependent manner or in a condition-independent manner when the second monomeric protein that includes the second C intein and the second modification protein that includes the second N intein coexist. The following collectively describes inteins that can be used as the first C intein, the first N intein, the second C intein, and the second N intein.

For example, in the present invention, the C intein and the N intein included in the intein may be designed based on an integrated intein, or a split-type intein may be used.

Examples of the integrated intein include gp41-1, gp41-8, NrdJ-1, SspGyrB, Cfa, and the like. Examples of the split-type intein include DnaE (dnaE-n and dnaE-c, which are catalyst subunits $\alpha$ of DNA polymerase III derived from cyanobacteria), MCM2 (SEQ ID NO: 3, Hut MCM-2 intein derived from archaea *Halorhabdus utahensis* DSM 12940, partial sequence of Uniprot: C7NUH7), and the like. In the amino acid sequence of SEQ ID NO: 3 below, regions surrounded by brackets correspond to the N intein, a region to be deleted, and the C intein from the N terminus side (Reference 9).

Reference 9: Ciragan A. et al., "Salt-inducible Protein Splicing in cis and trans by Inteins from Extremely Halophilic Archaea as a Novel Protein-Engineering Tool." J Mol Biol. 2016 Nov 20;428(23):4573-4588. doi: 10.1016/j.jmb.2016.10.006. Epub 2016 Oct 6. PMID: 27720988.

Amino acid sequence of MCM2 (SEQ ID NO: 3)

[CVTGDTLVQAGDGRRRIRELAGETAEAGSIEELPNGRTIRDVDIDVWTMTDDET

LTRRPVTAIHEYDAPETLYEVTLSTGEEVTVTPDHPFFIEQASGRVETPAEDLQPGDLVFVP

EGSAMATDG][GIAQIDTSSDRLGPAESGL][GDIGLRTIENVESVPDHDYDSVYDLTVEGT

HNFLANGMVVHN]

When the intein is MCM2, the C intein and the N intein derived from the MCM2 are the C intein having the amino acid sequence of SEQ ID NO: 4 below and the N intein having the amino acid sequence of SEQ ID NO: 5 below.

Amino acid sequence of C intein derived from MCM2 (SEQ ID NO: 4)

GIAQIDTSSDRLGPAESGL/GDIGLRTIENVESVPDHDYDSVYDLTVEGTHNFLAN

GMVVHN

Amino acid sequence of N intein derived from MCM2 (SEQ ID NO: 5)

CVTGDTLVQAGDGRRRIRELAGETAEAGSIEELPNGRTIRDVDIDVWTMTDDET

LTRRPVTAIHEYDAPETLYEVTLSTGEEVTVTPDHPFFIEQASGRVETPAEDLQPGDLVFVP

EGSAMATD

C intein derived from gp41-1 (SEQ ID NO: 6)
MMLKKILKIEELDERELIDIEVSGNHLFYANDILTHNSAG
N intein derived from gp41-1 (SEQ ID NO: 7)

SGYCLDLKTQVQTPQGMKEISNIQVGDLVLSNTGYNEVLNVFPKSKKKSYKITL

EDGKEIICSEEHLFPTQTGEMNISGGLKEGMCLYVKE

C intein derived from Cfa (SEQ ID NO: 8)
VKIISRKSLGTQNVYDIGVEKDHNFLLKNGLVASNC
N intein derived from Cfa (SEQ ID NO: 9)

CLSYDTEILTVEYGFLPIGKIVEERIECTVYTVDKNGFVYTQPIAQWHNRGEQEV

FEYCLEDGSIIRATKDHKFMTTDGQMLPIDEIFERGLDLKQVDGLP

The intein may be an intein (condition-dependent intein) that causes a binding reaction in a condition-dependent manner. It can also be said that, for example, the condition-dependent intein is an intein in which the binding reaction between inteins as described above and/or between the C intein and the N intein is caused under certain conditions. Examples of the condition-dependent intein include salt concentration-dependent inteins such as MCM2 and a combination of HsaPolII and HsaCDC21 derived from Halobacterium salinarum NRC-1 (ATCC 700922) (see Reference 9); temperature-dependent inteins (see Reference 10); and the like.
The salt concentration-dependent inteins are inteins in which the binding reaction between inteins as described above and/or between the C intein and the N intein is caused, that is, such a binding reaction has a greater likelihood of being caused, when the salt concentration relatively increases. The salt concentration-dependent inteins may be inteins in which the binding reaction is caused when the salt concentration reaches a certain concentration. It is assumed that the binding reaction of the salt concentration-dependent inteins is caused in a manner dependent on the ionic strength of the salt. Accordingly, a salt to be used to cause the binding reaction of the salt concentration-dependent inteins is not particularly limited and any salts can be used. Examples of the salts include chlorides such as sodium chloride, calcium chloride, potassium chloride, and magnesium chloride; sulfates such as sodium sulfate, calcium sulfate, potassium sulfate, and magnesium sulfate; nitrates such as sodium nitrate, calcium nitrate, potassium nitrate, and magnesium nitrate; carbonates such as sodium carbonate, calcium carbonate, potassium carbonate, and magnesium carbonate; phosphates such as sodium phosphate, calcium phosphate, potassium phosphate, and magnesium phosphate; and the like. When the salt concentration-dependent inteins are MCM2, the salt is preferably sodium chloride.

Reference 10: Zeidler, M. et al. "Temperature-sensitive control of protein activity by conditionally splicing inteins." Nat Biotechnol 22, 871-876 (2004). https://doi.org/10.1038/nbt979

A functional equivalent may be used as the intein as long as the protease activity and ligase activity thereof are maintained. The functional equivalent may be, for example, a polypeptide that has an amino acid sequence having 70% or more, 75% or more, 80% or more, 85% or more, 90% or more, 95% or more, 96% or more, 97% or more, 98% or more, or 99% or more identity to the reference amino acid sequence of the intein and that has the protease activity and the ligase activity. The functional equivalent may be, for example, a polypeptide that has an amino acid sequence consisting of the reference amino acid sequence of the intein with deletion, substitution, insertion, and/or addition of one or several amino acids and that has the protease activity and the ligase activity. The "one or several" amino acids above refer to, for example, 1 to 44 amino acids, 1 to 33 amino acids, 1 to 22 amino acids, 1 to 11 amino acids, 1 to 8 amino acids, 1 to 6 amino acids, 1 to 4 amino acids, 1 to 3 amino acids, 1 or 2 amino acids, or 1 amino acid. The substitution is preferably, for example, conservative substitution.

Although the first monomeric protein and the second monomeric protein may include one C intein or a plurality of C inteins, it is preferable that the first monomeric protein and the second monomeric protein include one C intein because they can be efficiently modified.

When the N terminus of the first monomeric protein is modified with the first addition component (i.e., the first addition component is added thereto), it is preferable to place the first C intein on the N terminus side of the first dimer formation domain. When the N terminus of the second monomeric protein is modified with the second addition component (i.e., the second addition component is added thereto), it is preferable to place the binding partner on the N terminus side of the second dimer formation domain.

The first C intein and the second C intein are configured to, for example, be capable of reacting with the first N intein and the second N intein, respectively. This allows the first modification protein to be configured to be capable of modifying (altering) the first monomeric protein, and the second modification protein to be configured to be capable of modifying (altering) the second monomeric protein. Accordingly, it is preferable that the first C intein and the second C intein are C inteins derived from different inteins. In this case, the first C intein and the second C intein include different C inteins. The first C intein and the first N intein are derived from, for example, the same intein. The second C intein and the second N intein are derived from, for example, the same intein.

In the first monomeric protein, the order of the first reaction tag and the first dimer formation domain can be determined, for example, in accordance with the position at which the first addition component is added in the first modification protein. When the first addition component is to be added to the N terminus side of the first dimer formation domain, the first reaction tag and the first dimer formation domain are arranged in this order from the N terminus side in the first monomeric protein. When the first addition component is to be added to the C terminus side of the first dimer formation domain, the first dimer formation domain and the first reaction tag are arranged in this order from the N terminus side in the first monomeric protein.

In the first reaction tag, the order of the binding tag and the first C intein can be determined, for example, in accordance with the position at which the first addition component is added in the first modification protein. When the first addition component is to be added to the N terminus side of the first dimer formation domain, the binding tag and the first C intein are arranged in this order from the N terminus side. In the second reaction tag, the order of the binding partner and the second C intein can be determined, for example, in accordance with the position at which the second addition component is added in the second modification protein. When the second addition component is to be added to the N terminus side of the second dimer formation domain, the binding partner and the second C intein are arranged in this order from the N terminus side.

In the first monomeric protein, the order of the binding tag, the first C intein, and the first dimer formation domain can be determined, for example, in accordance with the position at which the first addition component is added in the first modification protein. When the first addition component is to be added to the N terminus side of the first dimer formation domain, the binding tag, the first C intein, and the first dimer formation domain are arranged in this order from the N terminus side. In the second monomeric protein, the order of the binding partner, the second C intein, and the second dimer formation domain can be determined, for example, in accordance with the position at which the second addition component is added in the second modification protein. When the second addition component is to be added to the N terminus side of the second dimer formation domain, the binding partner, the second C intein, and the second dimer formation domain are arranged in this order from the N terminus side.

In the first monomeric protein, the binding tag, the first C intein, and the first dimer formation domain directly or indirectly bind to (are linked to) one another. When a heterodimeric protein is produced as the heterodimeric protein by linking a plurality of domains having only a natural amino acid sequence, it is preferable that the first C intein and the first dimer formation domain are directly linked together.

In the second monomeric protein, the binding partner, the second C intein, and the second dimer formation domain directly or indirectly bind to (are linked to) one another. When a heterodimeric protein is produced as the heterodimeric protein by linking a plurality of domains having only a natural amino acid sequence, it is preferable that the second C intein and the second dimer formation domain are directly linked together.

The "direct binding" means that an N-terminal or C-terminal amino acid of one polypeptide or domain forms a peptide bond together with a C-terminal or N-terminal amino acid of another polypeptide or domain and binds thereto. On the other hand, the "indirect binding" means that an N-terminal or C-terminal amino acid of one polypeptide or domain binds to a C-terminal or N-terminal amino acid of another polypeptide or domain via a linker peptide (peptide linker), that is, an N-terminal or C-terminal amino acid of one polypeptide or domain forms a peptide bond together with a C-terminal or N-terminal amino acid of the linker peptide and binds thereto while an amino acid at the other terminus of the linker peptide binds to an N-terminal or C-terminal amino acid of another polypeptide or domain. The linker peptide is composed of, for example, 5 to 15 amino acids. A known linker peptide can be used as the linker peptide, and specific examples thereof include GS linkers (GS, GGS, and GGGGS (SEQ ID NO: 10)), linker peptides formed by repeatedly linking GS linkers together ($[GS]_l$, $[GGS]_m$, or $[GGGGS]_n$ (l, m, n are integers greater than or equal to 2)), GGGSGG (SEQ ID NO: 11), and the like.

In the present invention, when the heterodimeric protein is a multispecific antibody, the dimeric protein includes, for example, the CH2 and CH3 regions in the Fc region of the immunoglobulin (antibody), or a portion or all of the hinge region of the immunoglobulin and the CH2 and CH3 regions in the Fc region of the immunoglobulin. In this case, the first dimer formation domain of the first monomeric protein and the second dimer formation domain of the second monomeric protein include, for example, amino acid sequences corresponding to the CH2 and CH3 regions in the Fc region of the immunoglobulin (antibody), or a portion or all of the hinge region of the immunoglobulin and the CH2 and CH3 regions in the Fc region of the immunoglobulin. The immunoglobulin is, for example, a human antibody, and preferably a human IgG.

The first monomeric protein may include, for example, other polypeptides such as a solubilization domain and a signal peptide on the N terminus side of the first reaction tag. The second monomeric protein may include, for example, other polypeptides such as a solubilization domain and a signal peptide on the N terminus side of the second reaction tag. The solubilization domain is preferably a polypeptide that, when fused to the polypeptide or protein, increases the expression level of the first monomeric protein or second monomeric protein expressed in a transformant, which will be described later, by at least 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, or 100% or more compared with expression of the polypeptide or protein without the solubilization domain. The solubilization domain is, for example, a protein or a partial polypeptide thereof. Specific examples of the solubilization domain include GST, MBP, thioredoxin, antibodies, altered antibodies such as a single-chain antibody, and the like.

When the first monomeric protein includes the solubilization domain, the first monomeric protein may include one solubilization domain or a plurality of solubilization domains. In the latter case, one type of solubilization domain or a plurality of types of solubilization domains may be used.

When the second monomeric protein includes the solubilization domain, the second monomeric protein may include one solubilization domain or a plurality of solubilization domains. In the latter case, one type of solubilization domain or a plurality of types of solubilization domains may be used.

The first monomeric protein and the second monomeric protein may include, for example, a purification tag for use to purify the first monomeric protein, the second monomeric protein, or the heterodimeric protein. For example, the affinity tag described above can be used as the purification tag. The purification tag can be added to, for example, at least one of the N terminus (side) and the C terminus (side) of the first dimer formation domain and at least one of the N terminus (side) and the C terminus (side) of the second dimer formation domain.

The reaction conditions in the formation step need only be conditions under which the first monomeric protein and the second monomeric protein can form a dimer, and can be determined as appropriate in consideration of the reaction conditions (binding conditions) for the binding tag and the binding partner and/or the reaction conditions (binding conditions) for the first dimer formation domain and the second dimer formation domain. Specifically, the reaction temperature in the formation step is, for example, 4 to 50°C or 10 to 45°C. The reaction time in the formation step is, for example, 0.1 to 24 hours or 0.5 to 12 hours. The reaction pH in the formation step is, for example, pH 4 to 10 or pH 5 to 9.

When the binding tag and the binding partner directly bind to each other, the reaction conditions in the formation step can be set to reaction conditions under which a binding between the binding tag and the binding partner is sufficiently formed. Specifically, when the SpyTag and the SpyCatcher are used as the binding tag and the binding partner, the formation step can be conducted, for example, at pH 5 to 8 at 4 to 37°C in the presence of a buffer solution. When a binding between the binding tag and the binding partner is generated through an enzyme reaction, the reaction conditions in the formation step can be determined, for example, based on the conditions under which an enzyme used in the enzyme reaction exhibits its activity.

When the first dimer formation domain and the second dimer formation domain directly bind to each other, the reaction conditions in the formation step can be set to reaction conditions under which a binding between the first dimer formation domain and the second dimer formation domain is sufficiently formed. Specifically, when the first dimer formation domain and the second dimer formation domain bind to each other via a disulfide bond, the reaction conditions in the formation step can be set to those under which the disulfide bond is not reduced.

When the heterodimeric protein is a multispecific antibody, the reaction conditions in the formation step may be conditions under which the first monomeric protein and the second monomeric protein can further form a disulfide between the hinge regions of the immunoglobulin.

Next, in the production step, as shown in FIGS. 1(C) and 1(D), the dimeric protein is reacted with the modification proteins for modifying the dimeric protein to produce (generate) a heterodimeric protein. Accordingly, it can also be said that the production step is a step of generating the heterodimeric protein by adding polypeptides or proteins derived from the modification proteins to the dimeric protein. Specifically, in the production step, the first N intein of the first modification protein reacts with the first C intein of the first monomeric protein, and thus the first addition component of the first modification protein is linked to the first monomeric protein, more specifically the N terminus side of the first dimer formation domain of the first monomeric protein (this step may also be referred to as a "first linking step" hereinafter). Also, in the production step, the second N intein of the second modification protein reacts with the second C intein of the second monomeric protein, and thus the second addition component of the second modification protein is linked to the second monomeric protein, more specifically the N terminus side of the first dimer formation domain of the first monomeric protein (this step may also be referred to as a "second linking step" hereinafter). Thus, in the production step, the dimeric protein can be converted into a heterodimeric protein composed of a protein that includes the first addition component and the first dimer formation domain in this order and a protein that includes the second addition component and the second dimer formation domain in this order.

It is preferable to cause the reaction in the production step in the presence of, for example, an aqueous solvent such as a buffer solution, physiological saline, or water. The dimeric protein, the first modification protein, and the second modification protein may be in the form of a solid or the form of a liquid, but the liquid form is preferable. When the dimeric protein, the first modification protein, and the second modification protein are in the form of a solid such as powder, it is preferable that the dimeric protein, the first modification protein, and the second modification protein are dispersed in an aqueous solvent such as a buffer solution, physiological saline, or water in advance. When the dimeric protein, the first modification protein, and the second modification protein are in the form of a liquid, the dimeric protein, the first modification protein, and the second modification protein may be diluted using an aqueous solvent such as a buffer solution, physiological saline, or water in advance. Specifically, for example, the reaction can be caused by mixing a solution containing the dimeric protein, a solution containing the first modification protein, and/or a solution containing the second modification protein.

In the production step, portions or all of the first linking step and the second linking step may be simultaneously conducted, or the first linking step and the second linking step may be separately conducted. When the first linking step and the second linking step are separately conducted, the order of the first linking step and the second linking step is not particularly limited, and the first linking step and the second linking step may be conducted in this order or in the reverse order.

The first modification protein includes the first addition component to be added to the first monomeric protein, and the first N intein capable of reacting with the first C intein. The first modification protein includes, for example, the first addition component and the first N intein in this order from the N terminus side. The second modification protein includes the second addition component to be added to the second monomeric protein, and the second N intein capable of reacting with the second C intein. The second modification protein includes, for example, the second addition component and the second N intein in this order from the N terminus side. Linking the first addition component of the first modification protein to the first N intein makes it possible to introduce the first addition component to the N terminus side of the first dimer formation domain when the first N intein and the first C intein react. As a result, in the first linking step, a fusion protein that includes the first dimer formation domain and the first addition component can be generated. Linking the second addition component of the second modification protein to the second N intein makes it possible to introduce the second addition component to the N terminus side of the second dimer formation domain when the second N intein and the second C intein react. As a result, in the second linking step, a fusion protein that includes the second dimer formation domain and the second addition component can be generated.

Regarding the descriptions of the first N intein in the first modification protein and the second N intein in the second modification protein, the descriptions above can be referred to. The first N intein and the second N intein are configured to, for example, be capable of reacting with the first C intein and the second C intein, respectively. This allows the first modification protein to be configured to be capable of modifying (altering) the first monomeric protein, and the second modification protein to be configured to be capable of modifying (altering) the second monomeric protein. Accordingly, it is preferable that the first N intein and the second N intein are N inteins derived from different inteins. In this case, the first N intein and the second N intein include different N inteins. The first C intein and the first N intein are derived from, for example, the same intein. The second C intein and the second N intein are derived from, for example, the same intein.

The first addition component and the second addition component are components to be added to the dimeric protein. The addition component can also be referred to as, for example, a "modifier". The first addition component and the second addition component each include one or more desired peptides, polypeptides, or proteins, and in addition, the desired peptides, polypeptides, or proteins may be modified. The first addition component and the second addition component are different addition components. With the production method of the present invention, using different addition components as the first addition component and the second addition component makes it possible to add the different addition components to one dimer formation domain and the other dimer formation domain included in the dimeric protein, thus making it possible to produce a heterodimeric protein. The following collectively describes addition components that can be used as the first addition component and the second addition component.

Examples of the addition components include proteins such as an altered antibody, a heavy chain antibody (VHH), a receptor, a cytokine, a chemokine, a toxin, an enzyme, a fluorescent protein, and a collagen-binding domain; peptides such as insulin; and the like.

The altered antibody is a protein that includes an antigen-binding fragment of the antibody or includes a polypeptide formed by linking the antigen-binding fragments via a linker peptide. Examples of the altered antibody include Fab, Fab', $F(ab')_2$, a single-chain antibody (scFv), tandem scFv, BiTE, Diabody, DART, TandAb, scDiabody, a heavy chain antibody (VHH), a variable domain of the VHH. The altered antibody may also be a protein formed by further linking an antigen-binding fragment of another antibody or the like to the protein that includes the antigen-binding fragment of the antibody or includes a polypeptide formed by linking the antigen-binding fragments via a linker peptide. In this case, the altered antibody can be configured as a protein that includes, for example, a portion or all of a domain other than the Fc region, more specifically a domain on the N terminus side relative to the Fc region, in DVD-IgG (Reference 5), scFv-(H)IgG (Reference 5), IgG(L)-scFv (Reference 5), scFv-(L)IgG (Reference 5), V(H)-IgG (Reference 5), IgG(L)-V (Reference 5), V(L)-IgG (Reference 5), 2scFv-IgG (Reference 5), scFv4-Ig (Reference 5), Zybody (Reference 5), scDiabody-CH3 (Reference 5), Diabody-CH3 (Reference 5), scDiabody-Fc (Reference 5), Diabody-Fc (Reference 5), or the like.

A target antigen of the altered antibody or the heavy chain antibody is not particularly limited and any antigen can be used. Examples of the target antigen include a tumor antigen, a viral antigen, a bacterial antigen, a parasitic antigen, an antigen involved in an autoimmune disease, a sugar chain antigen, a surface antigen of an immune cell such as a lymphocyte, and the like.

The surface antigen may be, for example, a CD (cluster of differentiation) antigen. The surface antigen may be such an antigen that an immune cell expressing the surface antigen is activated or its activation is suppressed when an altered antibody or heavy chain antibody binds to the surface antigen. Examples of the immune cell include a T cell, an NK cell, an NKT cell, a B cell, a macrophage, and the like. Examples of the antigen that activates the T cell include CD2, CD3, CD4, CD5, CD8α, CD8β, CD27, CD28, CD134 (OX40), CD137 (4-1BB), CD154, GITR, ICOS, and the like, among which CD3 is preferable. Examples of the CD3 include CD3γ, CD3δ, CD3ε, CD3ζ, CD3η, and the like, among which CD3ε is preferable. Examples of the antigen that suppresses the activation of the T cell include CTLA4, PD-1, LAG3, B7-H3, TIM3, TIGIT, and the like. Examples of the antigen that activates the NK cell include CD94/NKG2C, CD94/NKG2E, NKG2D/NKG2D, and the like. Examples of the antigen that suppresses the activation of the NK cell include KIR2DL1, KIR2DL2, KIR2DL3, KIR3DL1, KIR3DL2, KIR2DL4, KIR2DL5, KIR3DL3, and the like. Examples of the antigen that activates the NKT cell include the antigens that activate the T cell and the antigens that activate the NK cell. Examples of the antigen that suppresses the activation of the NKT cell include the antigens that suppress the activation of the T cell and the antigens that suppress the activation of the NK cell.

Examples of the receptor include a cytokine receptor such as a TNFα receptor; a chemokine receptor; a hormone receptor such as an insulin receptor; a receptor of a growth factor or proliferation factor such as an EGF receptor; and the like.

Examples of the cytokine and the chemokine include TNFs (Tumor Necrosis Factors) such as TNF-α and TNF-β; lymphotoxins; interleukins of IL-1 to IL-38 (e.g., IL-1α, IL-1β, IL-2, IL-3, IL-4, IL-5, IL-6, IL-7, IL-8, IL-10, IL-11, IL-12, IL-17, IL-18, IL-23, IL-33, and IL-36); chemokines; hematopoietic factors; cell growth factors; adipokines; growth factors such as VEGF; and the like.

Examples of the toxin include staphylococcal enterotoxin and the like.

Examples of the enzyme include luciferase, phosphatase, and the like.

Examples of the fluorescent protein include GFP (Green Fluorescent Protein), dsRED, and the like.

In the first modification protein, the first addition component and the first N intein directly or indirectly bind to (are linked to) each other. When a heterodimeric protein is produced as the heterodimeric protein by linking a plurality of domains having only a natural amino acid sequence, it is preferable that the first addition component and the first N intein are directly linked together.

In the second modification protein, the second addition component and the second N intein directly or indirectly bind to (are linked to) each other. When a heterodimeric protein is produced as the heterodimeric protein by linking a plurality of domains having only a natural amino acid sequence, it is preferable that the second addition component and the second N intein are directly linked together.

Regarding the direct or indirect binding of the first addition component and the second addition component, the descriptions above can be referred to.

In the present invention, when the heterodimeric protein is a multispecific antibody, the first addition component and the second addition component are, for example, polypeptides that include the altered antibody or the heavy chain antibody. Specifically, for example, when the heterodimeric protein is a bispecific antibody, the first addition component is a polypeptide with an amino acid sequence that includes the Fab region of a first antibody or the Fab region and a portion or all of the hinge region of the antibody. The second addition component is a polypeptide with an amino acid sequence that includes the Fab region of a second antibody or the Fab region and a portion or all of the hinge region of the antibody. The first antibody and the second antibody are antibodies that bind to different antigens or different epitopes. When the first addition component and the second addition component each include a portion of the hinge region of an antibody, the hinge regions included in the first addition component and the second addition component are set so as to form the entire hinge regions of the antibodies when linked to the first dimer formation domain and the second dimer formation domain. The altered antibody is, for example, an altered antibody derived from a human antibody, and preferably an altered antibody derived from a human IgG.

The first modification protein may include, for example, other polypeptides such as the solubilization domain and the signal peptide on the N terminus side. The second modification protein may include, for example, other polypeptides such as the solubilization domain and the signal peptide on the N terminus side.

When the first modification protein includes the solubilization domain, the first modification protein may include one solubilization domain or a plurality of solubilization domains. In the latter case, one type of solubilization domain or a plurality of types of solubilization domains may be used.

When the second modification protein includes the solubilization domain, the second modification protein may include one solubilization domain or a plurality of solubilization domains. In the latter case, one type of solubilization domain or a plurality of types of solubilization domains may be used.

The first modification protein and the second modification protein may include, for example, a purification tag for use to purify the first modification protein, the second modification protein, or the heterodimeric protein. For example, the affinity tag described above can be used as the purification tag. The purification tag can be added to, for example, at least one of the N terminus (side) and the C terminus (side) of the first addition component and at least one of the N terminus (side) and the C terminus (side) of the second addition component.

In the production step, the reaction conditions of the first linking step may be the same as or different from those of the second linking step. The reaction conditions of the first linking step can be determined in accordance with, for example, the type of the first C intein and the type of the first N intein, and more specifically, the reaction conditions can be determined in accordance with the conditions under which an active intein formed by the first C intein and the first N intein binding to each other exhibits enzymatic activity. The reaction conditions of the second linking step can be determined in accordance with, for example, the type of the second C intein and the type of the second N intein, and more specifically, the reaction conditions can be determined in accordance with the conditions under which an active intein formed by the second C intein and the second N intein binding to each other exhibits enzymatic activity. Specifically, the reaction temperature in the production step is, for example, 0 to 40°C, 4 to 37°C, or 4 to 30°C. The reaction time in the production step is, for example, 1 minute to 48 hours, 30 minutes to 48 hours, or 1 to 48 hours. The reaction pH in the production step is, for example, pH 5 to 10, pH 6 to 9, or pH 6.5 to 9.

The production method of the present invention may include a purification step of purifying the heterodimeric protein after the production step. For example, a common protein purification method such as chromatography can be utilized as the purification method in the purification step.

Thus, with the production method of the present invention, it is possible to produce a heterodimeric protein from a dimeric protein.

<Dimeric Protein>

Another aspect of the present invention provides a dimeric protein that can be favorably used to produce a heterodimeric protein. The protein (dimeric protein) of the present invention includes a dimeric protein having a reaction tag, wherein the dimeric protein having a reaction tag includes a first monomeric protein and a second monomeric protein, the first monomeric protein includes a first reaction tag and a first dimer formation domain capable of forming a dimer, the first reaction tag includes a binding tag and a first C intein capable of reacting with a first N intein, the second monomeric protein includes a second reaction tag and a second dimer formation domain capable of forming a dimer together with the first dimer formation domain in this order, the second reaction tag includes a binding partner capable of binding to the binding tag, and a second C intein capable of reacting with a second N intein, and the first monomeric protein and the second monomeric protein form a dimer. Using the dimeric protein of the present invention in combination with the first modification protein and the second modification protein makes it possible to favorably produce a heterodimeric protein.

<Monomeric Protein>

Another aspect of the present invention provides a monomeric protein that can be favorably used to produce the dimeric protein. The protein of the present invention includes a first monomeric protein, wherein the first monomeric protein includes a first reaction tag and a first dimer formation domain capable of forming a dimer in this order, the first reaction tag includes a binding tag capable of binding to a binding partner and a first C intein capable of reacting with a first N intein, the first monomeric protein is capable of forming a dimer together with a second monomeric protein, the second monomeric protein includes a second reaction tag and a second dimer formation domain capable of forming a dimer together with the first dimer formation domain, and the second reaction tag includes a binding partner capable of binding to the binding tag, and a second C intein capable of reacting with a second N intein. Also, the protein of the present invention includes a second monomeric protein, wherein the second monomeric protein includes a second reaction tag and a second dimer formation domain capable of forming a dimer in this order, the second reaction tag includes a binding partner capable of binding to a binding tag and a second C intein capable of reacting with a second N intein, the second monomeric protein is capable of forming a dimer together with a first monomeric protein, the first monomeric protein includes a first reaction tag and a first dimer formation domain capable of forming a dimer together with the second dimer formation domain in this order, and the first reaction tag includes a binding tag capable of binding to the binding partner, and a first C intein capable of reacting with a first N intein.

<Nucleic Acid>

Another aspect of the present invention provides a nucleic acid that can be used to synthesize the dimeric protein, the first monomeric protein, and/or the second monomeric protein. The nucleic acid of the present invention encodes the dimeric protein of the present invention, the first monomeric protein of the present invention, and/or the second monomeric protein of the present invention.

The nucleic acid of the present invention can be designed through replacement with corresponding codons based on the amino acid sequences of the dimeric protein of the present invention, the first monomeric protein of the present invention, and/or the second monomeric protein of the present invention. The base sequence of the nucleic acid of the present invention may be subjected to, for example, codon optimization.

The nucleic acid of the present invention may further encode the first modification protein and/or the second modification protein.

<Expression Vector>

Another aspect of the present invention provides an expression vector that can be used to synthesize the dimeric protein, the first monomeric protein, and/or the second monomeric protein. The expression vector of the present invention includes the nucleic acid of the present invention. With the expression vector of the present invention, it is possible to produce the dimeric protein of the present invention, the first monomeric protein of the present invention, and/or the second monomeric protein of the present invention (these proteins may also be referred to as "proteins of the present invention" hereinafter) using a genetic engineering technique.

The expression vector of the present invention is formed by, for example, inserting the nucleic acid of the present invention into an expression vector. The term "expression vector" means, for example, a nucleic acid molecule capable of transporting the inserted gene into a target such as a cell.

It is sufficient that the expression vector includes a polynucleotide encoding the dimeric protein of the present invention, the first monomeric protein of the present invention, and/or the second monomeric protein of the present invention so as to be capable of expressing the protein of the present invention encoded by the polynucleotide of the nucleic acid of the present invention, and the configuration thereof is not particularly limited. Regarding the dimeric protein of the present invention, the first monomeric protein of the present invention, and/or the second monomeric protein of the present invention, portions or all of them may be inserted into the same expression vector, or they may be inserted into separate expression vectors. When the dimeric protein of the present invention, the first monomeric protein of the present invention, and/or the second monomeric protein of the present invention are inserted into separate expression vectors, the expression vector of the present invention can be configured as an expression vector set containing an expression vector that includes the nucleic acid encoding the dimeric protein of the present invention, an expression vector that includes the nucleic acid encoding the first monomeric protein of the present invention, and/or an expression vector that includes the nucleic acid encoding the second monomeric protein of the present invention.

The expression vector may further include a nucleic acid encoding the first modification protein and/or the second modification protein. In this case, the nucleic acid encoding the first modification protein and/or the second modification protein may be inserted into the expression vector that includes the nucleic acid encoding the dimeric protein of the present invention, the first monomeric protein of the present invention, and/or the second monomeric protein of the present invention, or another expression vector.

The expression vector can be produced by, for example, inserting a polynucleotide encoding the protein of the present invention, that is, the nucleic acid of the present invention, into a skeleton vector (also referred to as a "basic vector" hereinafter). The type of the expression vector is not particularly limited and can be determined as appropriate in accordance with, for example, the type of the host.

Examples of the host include non-human hosts such as microorganisms, animal cells, and insect cells, or cultured cells thereof, isolated human cells or cultured cells thereof, mammalian cells, and the like. Examples of the prokaryotes include bacteria belonging to the genus Escherichia such as Escherichia coli, and bacteria belonging to the genus Pseudomonas such as Pseudomonas putida. Examples of the eukaryotes include yeasts such as Saccharomyces cerevisiae, and the like. Examples of the animal cells include an HEK293 cell, an Expi293F cell, a COS cell, a CHO cell, and the like, and examples of the insect cells include Sf9, Sf21, and the like.

Examples of the expression vector (basic vector) include viral vectors and non-viral vectors. When the host is transformed using a heat shock method as the introduction method, the expression vector is, for example, a binary vector or the like. Examples of the expression vector include pETDuet-1, pQE-80L, pUCP26Km, and the like. Examples of the expression vector for transformation of bacteria such as Escherichia coli include a pETDuet-1 vector (Novagen), pQE-80L (QIAGEN), pBR322, pB325, pAT153, pUC8, and the like. Examples of the expression vector for transformation of the yeasts include pYepSec1, pMFa, pYES2, and the like. Examples of the expression vector for transformation of the insect cells include pAc, pVL, and the like. Examples of the expression vector for transformation of the mammalian cells include pcDNA3.1, pcDNA3.4, pCAG, pCAGEN, pCDM8, pMT2PC, and the like.

It is preferable that the expression vector has a regulatory sequence that regulates, for example, the expression of the polynucleotide encoding the protein of the present invention and the expression of the protein of the present invention encoded by the polynucleotide for the protein of the present invention. Examples of the regulatory sequence include a promoter, a terminator, an enhancer, a polyadenylation signal sequence, a replication origin sequence (ori), and the like. The position of the regulatory sequence in the expression vector is not particularly limited. It is sufficient that the regulatory sequence is placed in the expression vector so as to, for example, be capable of functionally regulating the expression of the polynucleotide encoding the protein of the present invention and the expression of the protein of the present invention encoded thereby, and the regulatory sequence can be placed based on a known method. The regulatory sequence may be one originally included in the basic vector, or the regulatory sequence may be further inserted into the basic vector, or a regulatory sequence included in the basic vector may be replaced with another regulatory sequence.

The expression vector may further have, for example, a sequence encoding a selection marker. Examples of the selection marker include a drug-resistance marker, a fluorescent protein marker, an enzyme marker, a cell-surface receptor marker, and the like.

DNA, the regulatory sequence, and/or the sequence encoding the selection marker may be inserted into the expression vector, for example, using a method in which a restriction enzyme and a ligase are used, or using a commercially available kit or the like.

<Transformant and Method for Producing Transformant>

Another aspect provides a transformant capable of producing the protein of the present invention and a method for producing the transformant. The transformant of the present invention includes a nucleic acid encoding the protein of the present invention. With the transformant of the present invention, it is possible to favorably produce the protein of the present invention.

The method for producing a transformant according to the present invention includes a step of introducing the nucleic acid of the present invention into a host. With the method for producing a transformant according to the present invention, it is possible to produce the transformant above.

Regarding the nucleic acid encoding the protein of the present invention in the transformant of the present invention, the descriptions above of the nucleic acid encoding the protein of the present invention can be referred to. The expression vector of the present invention may be used as the nucleic acid of the present invention.

In the transformant of the present invention, the nucleic acid of the present invention is present as an exogenous molecule. Accordingly, the transformant of the present invention can be produced, for example, by introducing the nucleic acid of the present invention into the host.

The method for introducing the nucleic acid is not particularly limited and a known method can be used. The nucleic acid may be introduced, for example, using the expression vector. The introduction method can be determined as appropriate, for example, in accordance with the type of the host. Examples of the introduction method include an introduction method in which a gene gun such as a particle gun is used, a calcium phosphate method, a polyethylene glycol method, a lipofection method in which a liposome is used, an electroporation method, an ultrasonic nucleic acid introduction method, a DEAE-dextran method, a direct injection method in which a micro glass tube or the like is used, a hydrodynamic method, a cationic liposome method, a method in which an introduction auxiliary is used, a method in which an agrobacterium is used as a mediator, and the like. Examples of the liposome include Lipofectamine, a cationic liposome, and the like, and examples of the introduction auxiliary include atelocollagen, a nano particle, a polymer, and the like. When the host is a bacterium, for example, a method in which E. coli or Ps. putida is used as a mediator is particularly preferable. The polynucleotide encoding the protein of the present invention may be introduced into the host using, for example, the expression vector of the present invention.

<Method for Producing Protein>

Another aspect of the present invention provides a method for producing a dimeric protein that can be favorably used to produce a heterodimeric protein, or a monomeric protein that can be favorably used to produce the dimeric protein. The method for producing a protein according to the present invention includes an expression step of expressing the nucleic acid of the present invention, the expression vector of the present invention, and/or the expression vector set of the present invention. With the method for producing a protein according to the present invention, it is possible to produce the dimeric protein of the present invention, the first monomeric protein of the present invention, and/or the second monomeric protein of the present invention.

The protein of the present invention may be expressed using, for example, the expression vector of the present invention. A method for expressing the protein of the present invention is not particularly limited and a known method can be employed. For example, a host may be used, or a cell-free protein synthesis system may be used.

In the former case, it is preferable to, for example, use the host into which the protein of the present invention or the nucleic acid encoding the protein has been introduced and culture the host to express the protein of the present invention in the host. Thus, for example, introducing a nucleic acid encoding the protein of the present invention into a host makes it possible to produce a transformant that synthesizes the protein of the present invention, and the protein of the present invention can be synthesized by culturing the transformant.

The method for culturing the host is not particularly limited and can be determined as appropriate in accordance with the type of the host. A culture medium for use in the culture is not particularly limited and can be determined as appropriate in accordance with the type of the host.

In the latter case, it is preferable to express the polynucleotide for the protein of the present invention in a cell-free protein synthesis system. In this case, an expression vector may be used to express the polynucleotide for the protein of the present invention. The cell-free protein synthesis system is implemented through a known method using, for example, a cell extract, a buffer containing various components, and an expression vector into which a polynucleotide encoding the protein of the present invention is introduced, and for example, a commercially available reagent kit can be used.

The method for producing a protein according to the present invention may include, for example, a collection step of collecting the protein of the present invention. The protein of the present invention obtained in the collection step may be, for example, a crude product or a purified protein.

When the protein is collected from the culture solution, insoluble matter is removed in the collection step by, for example, filtering or centrifuging the culture supernatant. Then, in the collection step, the protein of the present invention can be obtained by, for example, subjecting the culture supernatant from which the insoluble matter have been removed to separation and purification using the following techniques in combination as appropriate: concentration with an ultrafilter; salting-out such as ammonium sulfate precipitation; dialysis; and chromatography with various columns such as an ion-exchange column and a gel-filtration column.

When the protein is collected from the transformant, for example, the transformant is homogenized through pressurization, ultrasonication, or the like in the collection step. The protein of the present invention can be obtained by removing insoluble matter from the obtained homogenate solution and subjecting the resulting solution to separation and purification as described above.

For example, the protein of the present invention obtained through the production method of the present invention may be used as a crude protein as it is, or may be partially purified and used as a partially purified protein, or may be purified to a single product and used as a purified protein.

In the production method of the present invention, powder of the protein of the present invention may be produced through, for example, freeze drying, vacuum drying, or spray drying. In this case, in the production method of the present invention, for example, the protein of the present invention may be dissolved in a buffer solution such as an acetate buffer solution, a phosphate buffer solution, a triethanolamine buffer solution, a tris-hydrochloric acid buffer solution, a GOOD's buffer (e.g., HEPES, PIPES, MES, MOPS, or the like), or the like in advance.

<Method for Screening Heterodimeric Protein>

Another aspect of the present invention provides a method for screening a heterodimeric protein that reacts with a desired target. The method for screening a target-reactive heterodimeric protein according to the present invention includes: a production step of producing a candidate heterodimeric protein by reacting a dimeric protein having a reaction tag with a modification protein for modifying the dimeric protein; a detection step of detecting a reaction between the candidate heterodimeric protein and a target by bringing the candidate heterodimeric protein into contact with the target; and a selection step of selecting the candidate heterodimeric protein used when the reaction is detected as a candidate of a substance that reacts with the target, wherein the production step is conducted using the production method of the present invention above. With the screening method of the present invention, it is possible to screen a heterodimeric protein that reacts with a desired target.

In the production step, the candidate heterodimeric protein can be obtained in the same manner as the production method of the present invention. The candidate heterodimeric protein is preferably an antibody such as a multispecific antibody or a bispecific antibody. When the candidate heterodimeric protein is an antibody, the antigen-binding domain of the antibody may be considered as, for example, a candidate antigen-binding domain that is evaluated for an ability to bind to a target, which will be described below.

The candidate heterodimeric protein may include a label. Examples of the label include fluorescent substances such as a fluorescent protein and a fluorescent dye; enzymes such as luciferase and phosphatase; and the like.

Next, in the detection step, the candidate heterodimeric protein is brought into contact with a target and a reaction between the candidate heterodimeric protein and the target is detected. In the detection step, the contact is achieved, for example, in the presence of the aqueous solvent above. The conditions for the contact are not particularly limited and can be determined in accordance with, for example, the type of the target. The temperature in the detection step is, for example, 4 to 37°C or 18 to 25°C. The duration of the detection step is, for example, 0 to 120 minutes or 30 to 60 minutes.

A desired target for screening the reactivity of the heterodimeric protein can be used as the target above. Regarding the target, for example, the examples of the target antigen can be referred to. During the contact, the target may be, for example, immobilized on a carrier or be in the free form. Examples of the carrier include a substrate, a bead, a container, and the like. Examples of the container include a microplate, a tube, and the like. When the target is a molecule capable of being expressed in a cell, the target may be a cell expressing the target.

Next, in the detection step, a reaction between the candidate heterodimeric protein and the target is detected. The reaction can be determined in accordance with the types of the candidate heterodimeric protein and the target. Specifically, for example, when the candidate heterodimeric protein includes an antibody such as a multispecific antibody or an antigen-binding fragment thereof, the reaction is, for example, a binding. When the candidate heterodimeric protein includes an enzyme, the reaction is, for example, a catalytic reaction. When the candidate heterodimeric protein includes a receptor, the reaction is, for example, a binding.

Then, in the detection step, detecting the presence of the reaction between the candidate heterodimeric protein and the target makes it possible to, for example, detect or analyze (qualitative analysis) the presence of the reactivity of the candidate heterodimeric protein to the target. Also, in the detection step, detecting the degree of the reaction between the candidate heterodimeric protein and the target makes it possible to, for example, detect or analyze (quantitative analysis) the intensity of the reaction of the candidate heterodimeric protein with the target.

When the reaction between the candidate heterodimeric protein and the target is not detected, it can be determined that the candidate heterodimeric protein does not react with the target, whereas, when the reaction is detected, it can be determined that the candidate heterodimeric protein reacts with the target.

A method for analyzing the reaction between the candidate heterodimeric protein and the target is not particularly limited and can be determined in accordance with the type of the reaction. When the candidate heterodimeric protein includes a label and the reaction is a binding, the reaction between the candidate heterodimeric protein and the target can be analyzed by, for example, detecting the label in a complex generated through a binding between the candidate heterodimeric protein and the target.

Then, in the selection step, the candidate heterodimeric protein used when the reaction is detected is selected as a candidate of a substance that reacts with the target. Thus, with the screening method of the present invention, it is possible to screen a candidate heterodimeric protein that is assumed to have a reactivity to the target.

Examples

Hereinafter, the present invention will be described in detail by way of examples, but the present invention is not limited to aspects described in the examples.

[Example 1]

It was confirmed that different addition components could be introduced into the dimeric protein using the production method of the present invention.

Specifically, in Example 1, a protein that includes a portion of the hinge region of a human IgG antibody and the CH2 and CH3 regions in the Fc region thereof was used as a model of the dimeric protein, and the Fab of a HER2 antibody and the Fab of an OKT3 antibody were used as models of the first addition component and the second addition component, respectively. Then, the first monomeric protein, the second monomeric protein, the first modification protein, and the second modification protein containing these regions as parts were prepared, and it was confirmed that the production method of the present invention could be used to introduce the Fab of a HER2 antibody (Herceptin) and the Fab of an OKT3 antibody into the protein with the Fc region and thus produce a bispecific antibody capable of binding to HER2 and CD3.

(1) Preparation of Antibody Fragment Proteins for Herceptin-OKT3 Bispecific Antibody

In order to prepare a Herceptin-OKT3 bispecific antibody, the following monomeric proteins and modification proteins were prepared.

- First monomeric protein: a protein (SpyTag-VHH) containing SpyTag (binding tag), gp41-1 C intein (first C intein), and the Fc region and the C-terminal region of the hinge region of a human IgG1
- Second monomeric protein: a protein (SpyCatcher-VHH) containing SpyCatcher (binding partner), cfa C intein (second C intein), and the Fc region and the C-terminal region of the hinge region of a human IgG1
- First modification protein: (CD3-Fab)

A complex of a protein (CD3-Fab-L) containing the VL and CL regions of an hOKT3 antibody and a protein (CD3-Fab-H) containing the VH and the CH1 region of the hOKT3 antibody, the N-terminal region of the hinge region of a human IgG1, and go41-1 N intein (first N intein)

- Second modification protein (Her2-Fab):

A complex of a protein (Her2-Fab-L) containing the VL and CL regions of a Herceptin antibody and a protein (Her2-Fab-C) containing the VH and the CH1 region of the Herceptin antibody, the N-terminal region of the hinge region of a human IgG1, and cfa N intein (second N intein)

A plasmid vector capable of expressing the CD3-Fab-H was constructed in accordance with the following procedure. First, the entire length of a synthetic gene (Eurofins Genomics K.K.) having a base sequence encoding a signal peptide, the VH-CH1 domain of OKT3 (SEQ ID NO: 12), the N-terminal partial sequence of the hinge region of a human IgG1, the gp41-1 N intein (SEQ ID NO: 7), a G1 linker, and a His tag was amplified using PCR. The obtained full-length synthetic gene was linked to an animal cell expression vector (pCAGEN) to construct a recombinant protein expression vector. In the expression vector, as the CD3-Fab-H region (SEQ ID NO: 13), the signal peptide, the VH-CH1 domain of OKT3, the N-terminal partial sequence of the hinge region, the gp41-1 N intein, the G1 linker, and the His tag were linked in this order from the N terminus to the C terminus as indicated using brackets.

CD3-Fab-H region (SEQ ID NO: 13)

[MEFGLSWLFLVAILKGVQC][QVQLVQSGGGVVQPGRSLRLSCKASGYTFTRYT MHWVRQAPGKGLEWIGYINPSRGYTNYNQKVKDRFTISRDNSKNTAFLQMDSLRPEDT GVYFCARYYDDHYSLDYWGQGTPVTVSSASTKGPSVFPLAPSSKSTSGGTAALGCLVKD YFPEPVTVSWNSGALTSGVHTFPAVLQSSGLYSLSSVVTVPSSSLGTQTYICNVNHKPSNT KVDKKVEPKSC][DKT][SGYCLDLKTQVQTPQGMKEISNIQVGDLVLSNTGYNEVLNVF PKSKKKSYKITLEDGKEIICSEEHLFPTQTGEMNISGGLKEGMCLYVKE][GGSGG][HHHH HH]

Next, a plasmid vector capable of expressing the CD3-Fab-L was constructed in accordance with the following procedure. First, the entire length of a synthetic gene (Eurofins Genomics K.K.) having a base sequence encoding a signal peptide and the VL-CL domain of OKT3 (SEQ ID NO: 14) was amplified using PCR. The obtained full-length synthetic gene was linked to an animal cell expression vector (pCAGEN) to construct a recombinant protein expression vector. In the expression vector, as the CD3-Fab-L region (SEQ ID NO: 15), the signal peptide and the VL-CL domain of OKT3 were linked in this order from the N terminus to the C terminus as indicated using brackets.

CD3-Fab-L region (SEQ ID NO: 15)

[MDFQVQIFSFLLISASVIISRG][DIQMTQSPSSLSASVGDRVTITCSASSSVSYMN WYQQTPGKAPKRWIYDTSKLASGVPSRFSGSGSGTDYTFTISSLQPEDIATYYCQQWSSN PFTFGQGTKLQITRTVA][APSVFIFPPSDEQLKSGTASVVCLLNNFYPREAKVQWKVDNA LQSGNSQESVTEQDSKDSTYSLSSTLTLSKADYEKHKVYACEVTHQGLSSPVTKSFNRGE C]

A plasmid vector capable of expressing the Her2-Fab-H was constructed in accordance with the following procedure. First, the entire length of a synthetic gene (Eurofins Genomics K.K.) having a base sequence encoding a signal peptide, the VH-CH1 domain of Herceptin (SEQ ID NO: 16), the N-terminal partial sequence of the hinge region of a human IgG1, the Cfa N intein (SEQ ID NO: 9), a G1 linker, and an His tag was amplified using PCR. The obtained full-length synthetic gene was linked to an animal cell expression vector (pCAGEN) to construct a recombinant protein expression vector. In the expression vector, as the Her2-Fab-H region (SEQ ID NO: 17), the signal peptide, the VH-CH1 domain of Herceptin, the N-terminal partial sequence of the hinge region, the Cfa N intein, the G1 linker, and the His tag were linked in this order from the N terminus to the C terminus as indicated using brackets.

Her2-Fab-H region (SEQ ID NO: 17)

[MEFGLSWLFLVAILKGVQC][EVQLVESGGGLVQPGGSLRLSCAASGFNIKDTYI HWVRQAPGKGLEWVARIYPTNGYTRYADSVKGRFTISADTSKNTAYLQMNSLRAEDTA VYYCSRWGGDGFYAVDYWGQGTLVTVSSASTKGPSVFPLAPSSKSTSGGTAALGCLVK DYFPEPVTVSWNSGALTSGVHTFPAVLQSSGLYSLSSVVTVPSSSLGTQTYICNVNHKPSN TKVDKKVEPKSC][DKT][CLSYDTEILTVEYGFLPIGKIVEERIECTVYTVDKNGFVYTQPI AQWHNRGEQEVFEYCLEDGSIIRATKDHKFMTTDGQMLPIDEIFERGLDLKQVDGLP][G GSGG][HHHHHH]

Next, a plasmid vector capable of expressing the Her2-Fab-L was constructed in accordance with the following procedure. First, the entire length of a synthetic gene (Eurofins Genomics K.K.) having a base sequence encoding a signal peptide and the VL-CL domain of Herceptin (SEQ ID NO: 18) was amplified using PCR. The obtained full-length synthetic gene was linked to an animal cell expression vector (pCAGEN) to construct a recombinant protein expression vector. In the expression vector, as the Her2-Fab-L region (SEQ ID NO: 19), the signal peptide and the VL-CL domain of Herceptin were linked in this order from the N terminus to the C terminus as indicated using brackets.

Her2-Fab-L region (SEQ ID NO: 19)

[METPAQLLFLLLLWLPESTG][DIQMTQSPSSLSASVGDRVTITCRASQDVNTAVA WYQQKPGKAPKLLIYSASFLYSGVPSRFSGSRSGTDFTLTISSLQPEDFATYYCQQHYTTP PTFGQGTKVEI][KRTVAAPSVFIFPPSDEQLKSGTASVVCLLNNFYPREAKVQWKVDNAL QSGNSQESVTEQDSKDSTYSLSSTLTLSKADYEKHKVYACEVTHQGLSSPVTKSFNRGEC ]

A plasmid vector capable of expressing the SpyCatcher-VHH was constructed in accordance with the following procedure. First, the entire length of a synthetic gene (Eurofins Genomics K.K.) having a base sequence encoding a signal peptide, Ia1 (single-chain antibody (solubilization domain), SEQ ID NO: 20), a G1 linker, SpyCatcher (SEQ ID NO: 1), a G1 linker, Cfa C intein (SEQ ID NO: 8), the C-terminal partial sequence of the hinge region of a human IgG1, and the CH2 and CH3 regions of the Fc region of a human IgG (SEQ ID NO: 21) was amplified using PCR. The obtained full-length synthetic gene was linked to an animal cell expression vector (pCDNA3.4) to construct a recombinant protein expression vector. In the expression vector, as the SpyCatcher VHH region (SEQ ID NO: 22), the signal peptide, the Ia1, the G1 linker, the SpyCatcher, the G1 linker, the Cfa C intein, the C-terminal partial sequence of the hinge region of the human IgG1, and the CH2 and CH3 regions of the Fc region of the human IgG were linked in this order from the N terminus to the C terminus as indicated using brackets.

Fc region (SEQ ID NO: 21)

APELLGGPSVFLFPPKPKDTLMISRTPEVTCVVVDVSHEDPEVKFNWYVDGVEV HNAKTKPREEQYNSTYRVVSVLTVLHQDWLNGKEYKCKVSNKALPAPIEKTISKAKGQP REPQVYTLPPSRDELTKNQVSLTCLVKGFYPSDIAVEWESNGQPENNYKTTPPVLDSDGS FFLYSKLTVDKSRWQQGNVFSCSVMHEALHNHYTQKSLSLSPGK

Ia1 (SEQ ID NO: 20)

QVQLQESGGGLVQAGGSLLLSCAASGRTFSSYAMGWFRQAPGKEREFVAAINW SGGSTSYADSVKGRFTISRDNTKNTVYLQMNSLKPEDTAAFYCAATYNPYSRDHYFPRM TTEYDYWGQGTQVTVSS

SpyCatcher-VHH region (SEQ ID NO: 22)

[MEFGLSWLFLVAILKGVQC][QVQLQESGGGLVQAGGSLLLSCAASGRTFSSYA MGWFRQAPGKEREFVAAINWSGGSTSYADSVKGRFTISRDNTKNTVYLQMNSLKPEDT AAFYCAATYNPYSRDHYFPRMTTEYDYWGQGTQVTVSS][GGSGG][DSATHIKFSKRDE DGKELAGATMELRDSSGKTISTWISDGQVKDFYLYPGKYTFVETAAPDGYEVATAITFTV NEQGQVTVNG][GGSGG][VKIISRKSLGTQNVYDIGVEKDHNFLLKNGLVASNC][FNASY TCPPCP][APELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKAKG QPREPQVYTLPPSRDELTKNQVSLTCLVKGFYPSDIAVEWESNGQPENNYKTTPPVLDSD GSFFLYSKLTVDKSRWQQGNVFSCSVMHEALHNHYTQKSLSLSPGK]

A plasmid vector capable of expressing the SpyTag-VHH was constructed in accordance with the following procedure. First, the entire length of a synthetic gene (Eurofins Genomics K.K.) having a base sequence encoding a signal peptide, cAbGFP4 (single-chain antibody (solubilization domain), SEQ ID NO: 23), a G1 linker, SpyTag (SEQ ID NO: 2), gp41-1 C intein (SEQ ID NO: 6), the C-terminal partial sequence of the hinge region of a human IgG1, and the CH2 and CH3 regions of the Fc region of a human IgG was amplified using PCR. The obtained full-length synthetic gene was linked to an animal cell expression vector (pCDNA3.4) to construct a recombinant protein expression vector. In the expression vector, as the SpyTag-VHH region (SEQ ID NO: 24), the signal peptide, the cAbGFP4, the G1 linker, the SpyTag, the gp41-1 C intein, the C-terminal partial sequence of the hinge region of the human IgG1, and the CH2 and CH3 regions of the Fc region of the human IgG were linked in this order from the N terminus to the C terminus as indicated using brackets.

cAbGFP4 (SEQ ID NO: 23)

QVQLVESGGALVQPGGSLRLSCAASGFPVNRYSMRWYRQAPGKEREWVAGMS SAGDRSSYEDSVKGRFTISRDDARNTVYLQMNSLKPEDTAVYYCNVNVGFEYWGQGTQ VTVSS

SpyTag-VHH region (SEQ ID NO: 24)

[MEFGLSWLFLVAILKGVQC][QVQLVESGGALVQPGGSLRLSCAASGFPVNRYS MRWYRQAPGKEREWVAGMSSAGDRSSYEDSVKGRFTISRDDARNTVYLQMNSLKPED TAVYYCNVNVGFEYWGQGTQVTVSS][GGSGG][AHIVMVDAYKPTK][GGSGG][MMLK KILKIEELDERELIDIEVSGNHLFYANDILTHNSAG][ASYTCPPCP][APELLGGPSVFLFPPK PKDTLMISRTPEVTCVVVDVSHEDPEVKFNWYVDGVEVHNAKTKPREEQYNSTYRVVS VLTVLHQDWLNGKEYKCKVSNKALPAPIEKTISKAKGQPREPQVYTLPPSRDELTKNQV SLTCLVKGFYPSDIAVEWESNGQPENNYKTTPPVLDSDGSFFLYSKLTVDKSRWQQGNVF SCSVMHEALHNHYTQKSLSLSPGK]

Next, Expi293F cells were transfected with the recombinant protein plasmid expression vectors. In the transfection, a combination of the CD3-Fab-H expression vector and the CD3-Fab-L expression vector, a combination of the Her2-Fab-H expression vector and the Her2-Fab-L expression vector, or a combination of the SpyCatcher-VHH expression vector and the SpyTag-VHH expression vector was introduced into the cells. The obtained transformant was cultured using an HE400 culture medium in a $CO_2$ incubator at 37°C for 7 days to express a recombinant protein (SpyTag-VHH + SpyCatcher-VHH (Fc dimer), CD3-Fab-L + CD3-Fab-H (CD3-Fab), Her2-Fab-L + Her2-Fab-H (Her2-Fab)) into the supernatant of the culture medium.

Next, the culture supernatant containing the recombinant protein after the culture was collected.

(2) Purification of CD3-Fab and Her2-Fab

The culture supernatant was centrifuged at 1500 rpm for 5 minutes. After a reagent solution was added to the supernatant after the centrifugation such that the final concentration of NaCl was 500 mmol/l and the final concentration of imidazole was 10 mmol/l, the resultant solution was charged into an Ni-NTA column (manufactured by FUJIFILM Corporation) with a column volume of 3 ml that had been equilibrated with Wash Buffer (500 mmol/l NaCl, 50 mmol/l Tris-HCl, 10 mmol/l imidazole), and the column was washed with 30 ml of Wash Buffer and was then eluted with 15 ml of Elute Buffer (500 mmol/l NaCl, 50 mmol/l Tris-HCl, 500 mmol/l imidazole). The eluted fraction collected was dialyzed in a dialysis buffer (150 mmol/l NaCl, 50 mmol/l HEPES).

(3) Purification of Fc dimer

The culture supernatant was centrifuged at 1500 rpm for 5 minutes. The supernatant was charged into a protein A column (manufactured by SUPrA) with a column volume of 1 ml that had been equilibrated with Wash Buffer (50 mmol/l sodium phosphate buffer solution, pH 7.5), and the column was washed with 10 ml of Wash Buffer and was then eluted with 5 ml of Elute Buffer (20 mmol/l sodium citrate, 100 mmol/l NaCl, pH 3.0). When Elute Buffer was allowed to flow through the column, the eluted solution was poured into 5 ml of 100 mmol/l sodium phosphate buffer solution (pH 7.5) and was thus neutralized. The eluted fraction collected was dialyzed in a dialysis buffer (150 mmol/l NaCl, 50 mmol/l HEPES).

Each of the obtained protein samples was diluted with ultrapure water, and the absorbances at 280 nm and 330 nm were measured using an ultraviolet-visible spectrophotometer (UV-1800, manufactured by SHIMADZU Corporation). The molar concentration of the protein in each protein sample was calculated using the following formula (1).

$$C=(A280-A320)/(l\times\varepsilon)\times(\text{dilution factor}) ... (1)$$

C: Molar concentration of protein
A280: Absorbance at 280 nm
A320: Absorbance at 320 nm
l: Cell length
$\varepsilon$: Molar extinction coefficient of protein

(4) Production of Bispecific Antibody

A Herceptin-OKT3 bispecific antibody was produced using the SpyTag-VHH + SpyCatcher-VHH (Fc dimer), the CD3-Fab, and the Her2-Fab obtained in Example 1(1) above. Specifically, the PTS reaction was conducted to react the SpyTag-VHH + SpyCatcher-VHH, the CD3-Fab, and the Her2-Fab, and thus a Herceptin-Okt3 bispecific antibody was obtained. 2 µl of proteins with the antibody fragments obtained in Example 1(1) and Example 1(2) above (dissolved in 150 mmol/l NaCl, 50 mmol/l HEPES (pH 7.6), and 1 mmol/l TCEP) was added to a microtube to prepare a pre-PTS-reaction sample. After the preparation, the pre-PTS-reaction sample was left to stand in an incubator at 37°C for 24 hours, and thus the PTS reaction was conducted. After the PTS reaction, a post-PTS-reaction sample was obtained.

(5) Examination of Bispecific Antibody Binding by Bindings of Antibody Fragments

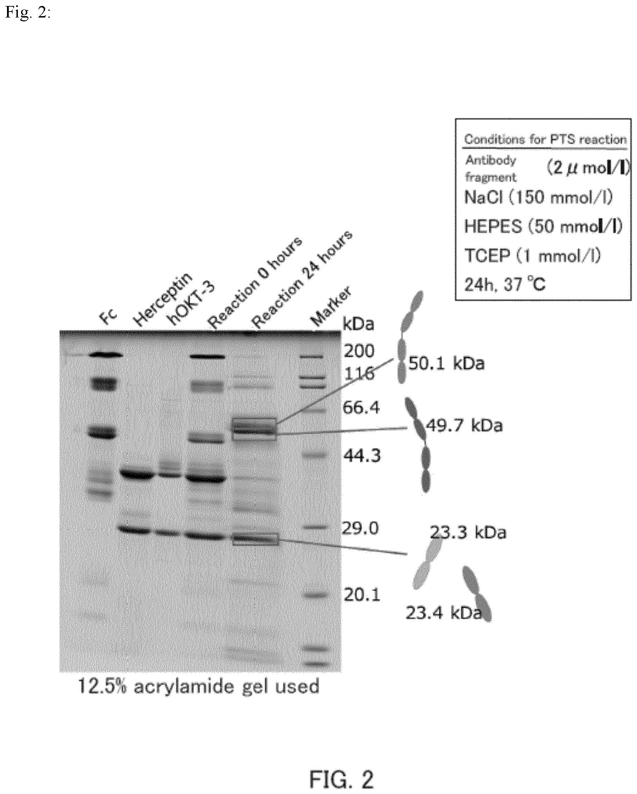

It was examined using SDS-PAGE whether a Herceptin-OKT3 bispecific antibody was produced in the post-PTS-reaction samples obtained in Example 1(4). Specifically, 10 µl of 5×SDS buffer was added to 40 µl of the post-PTS-reaction sample, the Fc region (the complex of the SpyTag-VHH and SpyCatcher-VHH), the Herceptin-Fab (Fab composed of the Her2-Fab-L and the Her2-Fab-H, Herceptin), the CD3-Fab (Fab composed of the CD3-Fab-L and the CD3-Fab-H, hOKT3), or the pre-PTS-reaction sample, followed by suspension. After the suspension, heat treatment was conducted at 95°C for 5 minutes. After the heat treatment, the samples were applied in the wells of 12.5% polyacrylamide gel, and electrophoresis was conducted at 150 to 200 V. The Fc region, the Herceptin, the hOKT3, the pre-PTS-reaction sample, the post-PTS-reaction sample, and the marker were applied on the 12.5% polyacrylamide gel. After the electrophoresis, the polyacrylamide gel was stained with a CBB (Coomassie Brilliant Blue) staining solution for 5 minutes, and destained with a destaining solution. FIG. 2 shows the results.

FIG. 2 is a photograph showing proteins before and after the PTS reaction and the antibody fragments. In FIG. 2, the types of samples are indicated on the upper side of the photograph, and the molecular weight (kDa) is shown on the right side of the photograph. In FIG. 2, the lanes correspond to, from the left side, the Fc region, the Herceptin, the hOKT3, the pre-PTS-reaction sample (Reaction 0 hours), the post-PTS-reaction sample (Reaction 24 hours), and the marker. As shown in FIG. 2, in the case of the post-PTS reaction sample, bands indicating the four proteins included in the antibodies were detected. It was found from these results that, in the post-PTS-reaction sample, the Herceptin-OKT3 bispecific antibody was produced through the PTS reaction.

(6) Purification of Bispecific Antibody

Next, the post-PTS-reaction sample obtained in Example 1(4) was purified. Specifically, after being purified using a column for purification of a protein having an Fc region, the sample was purified using a column for purification of a protein having a Fab region. First, a protein having an Fc region was purified using a KanCapA column. After a KanCapA column (manufactured by KANEKA Corporation, column volume of 500 μl) was equilibrated with a TBS buffer (150 mmol/l NaCl and 50 mmol/l Tris-HCl (pH 7.5)), the post-PTS-reaction sample obtained in Example 1(4) above was loaded onto the column, and a flow-through fraction was collected. After the collection, 20 ml of the TBS buffer was loaded, and then a washed fraction was collected. Furthermore, 2.8 ml of an Elute buffer (100 mmol/l Glycine-HCl (pH 2.8)) was loaded, and an eluted fraction was collected. In order to neutralize the eluted fraction, the eluted fraction was collected in a collection tube in which 1.2 ml of 1 mol/l Tris-HCl (pH 9.0) had been placed in advance. Then, the eluted fraction collected was placed in a transparent membrane (semipermeable membrane) and dialyzed using a dialysis buffer (1×PBS). Next, a protein having a Fab region in the post-dialysis protein sample was purified. After a KanCapL column (manufactured by KANEKA Corporation, column volume of 500 μl) was equilibrated with 1×PBS, the post-dialysis protein sample was loaded, and a flow-through fraction was collected. After the collection, 10 ml of 1×PBS was passed through the column, and a washed fraction was collected. Next, 7 ml of an Elute buffer (100 mmol/l Glycine-HCl (pH 2.8)) was loaded, and an eluted fraction was collected. In order to neutralize the eluted fraction, the eluted fraction was collected in a collection tube in which 3 ml of 1 mol/l Tris-HCl (pH 9.0) had been placed in advance. Then, the eluted fraction collected was placed in a transparent membrane (semipermeable membrane) and dialyzed using the dialysis buffer (1×PBS). After the dialysis, the sample was concentrated using a concentration tube. A purified sample was obtained through the concentration.

(7) Examination of Bispecific Antibody after Purification

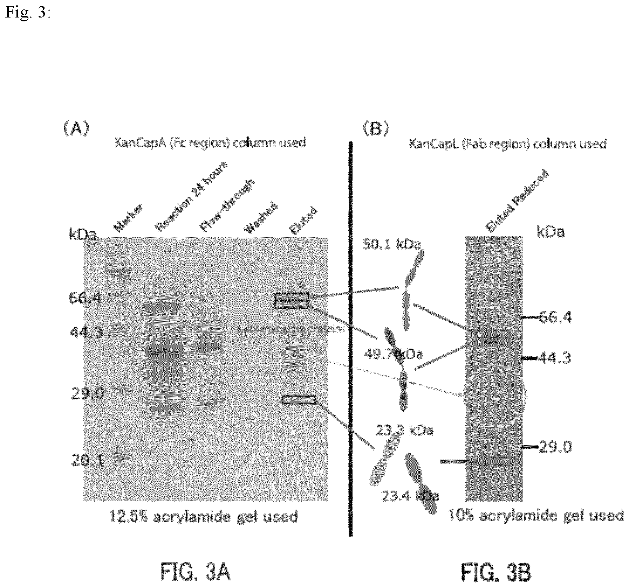

It was examined using SDS-PAGE whether the purified sample obtained in Example 1(6) above contained the Herceptin-Okt3 bispecific antibody. Specifically, 10 μl of 5×SDS buffer was added to the post-PTS-reaction sample, the eluted fraction purified using the KanCapA column, the flow-through fraction, or the washed fraction (each 40 μl), and the purified sample purified using the KanCapL column, followed by suspension. After the suspension, heat treatment was conducted at 95°C for 5 minutes. After the heat treatment, the samples were applied in the wells of 12.5% or 10% polyacrylamide gel, and electrophoresis was conducted at 150 to 200 V. The marker, the post-PTS-reaction sample, the flow-through fraction, the washed fraction, and the eluted fraction were applied on the 12.5% polyacrylamide gel. The purified sample was applied to the 10% acrylamide gel. After the electrophoresis, the polyacrylamide gels were stained with a CBB (Coomassie Brilliant Blue) staining solution for 5 minutes, and destained with a destaining solution. FIG. 3 shows the results.

FIG. 3 shows photographs illustrating proteins after the PTS reaction and column purification. FIG. 3(A) is a photograph illustrating proteins before and after the purification with the KanCapA column, and FIG. 3(B) is a photograph illustrating proteins in the purified sample purified using the KanCapL column. In FIG. 3(A), the types of samples are indicated on the upper side of the photograph, and the molecular weight (kDa) is shown on the left side of the photograph. In FIG. 3(B), the types of samples are indicated on the upper side of the photograph, and the molecular weight (kDa) is shown on the left side of the photograph. In FIG. 3(A), the lanes correspond to, from the left side, the marker, the post-PTS-reaction sample (Reaction 24 hours), the flow-through fraction (Flow-through), the washed fraction (Washed), and the eluted fraction (Eluted). As shown in FIG. 3(A), in the eluted fraction, bands of the antibody fragments of interest were detected, whereas bands of contaminating proteins were also detected. Meanwhile, as shown in FIG. 3(B), in the purified sample purified using the KanCapL column, bands of the antibody fragments of interest were detected, and bands of contaminating proteins were not detected. It was found from these results that only the target Herceptin-Okt3 bispecific antibody could be purified by conducting two-step column purification.

(8) Examination of Singularity of Purified Bispecific Antibody

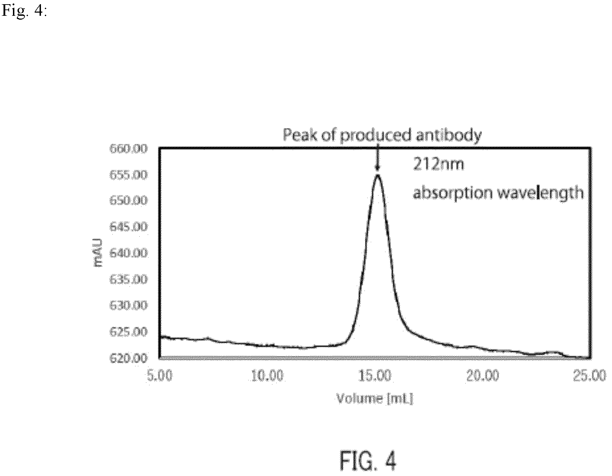

It was examined using gel filtration chromatography whether a Herceptin-Okt3 bispecific antibody was only a component of the Herceptin-Okt3 bispecific antibody purified in Example 1(6) above. Specifically, the protein purified in Example 1(6) above was passed through a gel filtration chromatography column (Superdex 200 increase 30/100 GL, manufactured by GE HealthCare) at 0.5 mL/min, and the absorbance at 212 nm was measured at room temperature (about 25°C). In the measurement above, $1 \times$PBS was used as a buffer. FIG. 4 shows the results.

FIG. 4 is a graph illustrating a protein elution pattern of gel filtration chromatography. In FIG. 4, the horizontal axis indicates the exclusion volume (ml), and the vertical axis indicates the absorbance. It was found that, as shown in FIG. 4, the purified protein obtained using the method of the present disclosure exhibited a single peak at 15 ml at an absorption wavelength of 212 nm. It was found from these results that the purified Herceptin-Okt3 bispecific antibody obtained using the method of the present disclosure contained a single component.

(9) Evaluation of Activity against CD3-Positive Cell

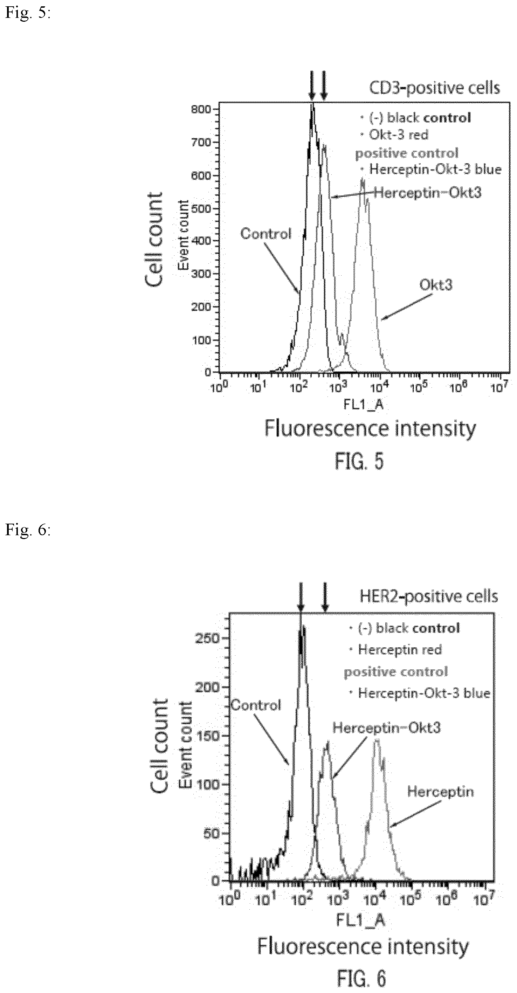

It was examined using flow cytometry whether the Herceptin-Okt3 bispecific antibody obtained in Example 1(6) above bound to a Her2- or CD3-expressing cell. Specifically, an HPB-ALL strain overexpressing CD3 on the cell membrane surface was used, and measurement was conducted using a flow cytometer. An equivalent amount of the HPB-ALL that had been cultured in a T75 flask was transferred to two 15-ml Falcon tubes and centrifuged at 1500 rpm for 5 minutes. After the centrifugation, the supernatant was aspirated using an aspirator, and a necessary amount of $1 \times$PBS was added and suspended to obtain a cell suspension of $1 \times 10^6$ cells/ml. Thereafter, 1 ml of the suspension was dispensed into three microtubes (a) to (c) and diluted with $1 \times$PBS. After the dilution, the two microtubes (a) and (b) were centrifuged at 2000 rpm at 25°C for 7 minutes. After the centrifugation, the supernatant was aspirated using an aspirator, and 1 ml of $1 \times$PBS was added. After the addition, the two microtubes (a) and (b) were centrifuged again at 2000 rpm at 25°C for 7 minutes. After the centrifugation, the supernatant was aspirated using an aspirator, $1 \times$PBS and the Herceptin-Okt3 bispecific antibody purified in Example 1(6) above were added to one of the microtubes, microtube (b), such that the final concentration of the Herceptin-Okt3 bispecific antibody was 0.05 $\mu$mol/l, followed by inversion mixing. After the inversion mixing, the microtube was left to stand for 20 minutes. After the standing, the tube was centrifuged at 2000 rpm at 25°C for 7 minutes. Thereafter, 1 $\mu$l of OKT3-FITC (manufactured by COSMO BIO Co., Ltd.) and 1 ml of $1 \times$PBS were added to the microtube (a) and 1 $\mu$l of anti-Fc-FITC (manufactured by Abcam Limited) and 1 ml of $1 \times$PBS were added to the microtube (b), followed by inversion mixing. After the inversion mixing, the microtubes were left to stand for 20 minutes. After the standing, the tubes were centrifuged at 2000 rpm at 25°C for 7 minutes. After the centrifugation, the supernatant was aspirated using an aspirator, and 1 ml of $1 \times$PBS was added. After the addition, the tubes were centrifuged again at 2000 rpm at 25°C for 7 minutes. After the centrifugation, the supernatant was aspirated using an aspirator, and 1 ml of $1 \times$PBS was added and suspended. After the suspension, the obtained suspensions were sterilized using a mesh filter. After the measurement conditions were set, a cell analyzer RF-500 (manufactured by Sysmex Corporation) was used to measure the negative control (c), the positive control (a), and the sample (b) in this order. After the measurement, FCSalyzer was used to show the measurement results graphically. FIG. 5 shows the results.

FIG. 5 shows a graph illustrating binding of the bispecific antibody to CD3-positive cells measured through flow cytometry. In FIG. 5, the horizontal axis indicates the fluorescence intensity, and the vertical axis indicates the cell count. As shown in FIG. 5, it was found that, when the Herceptin-Okt3 bispecific antibody obtained using the method of the present disclosure was added to CD3-positive cells, the fluorescent intensity was enhanced compared with the case where it was not added to CD3-positive cells. It was found from these results that the Herceptin-Okt3 bispecific antibody obtained using the production method of the present invention bound to CD3-positive cells.

(10) Evaluation of Activity against HER2-Positive Cell

It was examined using flow cytometry whether the Herceptin-Okt3 bispecific antibody obtained in Example 1(6) above bound to a breast cancer cell. Specifically, an SK-BR-3 strain overexpressing HER2 on the cell membrane surface was used, and measurement was conducted using a flow cytometer. The SK-BR-3 that had been cultured in a T75 flask was washed with 5 ml of $1 \times$PBS / 5 mmol/l EDTA after the supernatant was aspirated using an aspirator. After the washing, the supernatant was aspirated using an aspirator, and 5 ml of trypsin was added. Thereafter, the mixture was left to stand in an incubator at 37°C for 5 minutes. After the standing, the flask was lightly tapped. After the tapping, a DMEM culture medium was added to a total volume of 10 ml, and the cells were dissociated from the flask through suspension to obtain a cell suspension. Next, the cell suspension was transferred to a 15-ml Falcon tube and was centrifuged at 1500 rpm for 5 minutes. After the centrifugation, the supernatant was aspirated using an aspirator, and a necessary amount of $1 \times$PBS was added and suspended to obtain a cell suspension. Thereafter, 1 ml of the suspension containing $1 \times 10^6$ cells was dispensed into three microtubes (d) to (f) and diluted with $1 \times$PBS. After the dilution, the two microtubes (d) and (e) were centrifuged at 2000 rpm at 25°C for 7 minutes. After the centrifugation, the supernatant was aspirated using an aspirator, and 1 ml of 1×PBS was added. After the addition, the two microtubes (d) and (e) were centrifuged again at 2000 rpm at 25°C for 7 minutes. After the centrifugation, the supernatant was aspirated using an aspirator. After the aspiration, 1 μl of 5 mg/ml Herceptin (manufactured by Chugai Pharmaceutical Co., Ltd.) and 1 ml of 1×PBS were added to one of the microtubes, microtube (d), followed by inversion mixing. In parallel with this, 1 ml of 1×PBS and the Herceptin-Okt3 bispecific antibody purified in Example 1(6) above were added to one of the microtubes, microtube (e), such that the final concentration of the Herceptin-Okt3 bispecific antibody was 0.1 μmol/l, followed by inversion mixing. After the inversion mixing, the two microtubes (d) and (e) were left to stand for 20 minutes. After the standing, the two microtubes (d) and (e) were centrifuged at 2000 rpm at 25°C for 7 minutes. After the centrifugation, the supernatant was aspirated using an aspirator, and 1 ml of 1×PBS was added. After the addition, the two microtubes (d) and (e) were centrifuged again at 2000 rpm at 25°C for 7 minutes. After the centrifugation, the supernatant was aspirated using an aspirator, and 1 μl of anti-Fc-FITC (manufactured by Abcam Limited) and 1 ml of 1×PBS were added to the two microtubes (d) and (e), followed by inversion mixing. After the inversion mixing, the microtubes were left to stand for 20 minutes. After the standing, the two microtubes (d) and (e) were centrifuged at 2000 rpm at 25°C for 7 minutes. After the centrifugation, the supernatant was aspirated using an aspirator, and 1 ml of 1×PBS was added. After the addition, the two microtubes (d) and (e) were centrifuged again at 2000 rpm at 25°C for 7 minutes. After the centrifugation, the supernatant was aspirated using an aspirator, and 1 ml of 1×PBS was added and suspended. After the suspension, the obtained suspensions were sterilized using a mesh filter. After the measurement conditions were set, a cell analyzer RF-500 (manufactured by Sysmex Corporation) was used to measure the negative control (f), the positive control (d), and the sample (e) in this order. After the measurement, FCSalyzer was used to show the measurement results graphically. FIG. 6 shows the results.

FIG. 6 shows a graph illustrating binding of the bispecific antibody to HER2-positive cells measured through flow cytometry. In FIG. 6, the horizontal axis indicates the fluorescence intensity, and the vertical axis indicates the cell count. As shown in FIG. 6, it was found that, when the Herceptin-Okt3 bispecific antibody obtained using the production method of the present invention was added to HER2-positive cells, the fluorescent intensity was enhanced compared with the case where it was not added to HER2-positive cells. It was found from these results that the Herceptin-Okt3 bispecific antibody obtained using the method of the present disclosure bound to Her2-positive breast cancer cells.

(11) Evaluation of Maintained Activity against HER2-Positive Cell Conducted through Surface Plasmon Resonance Method

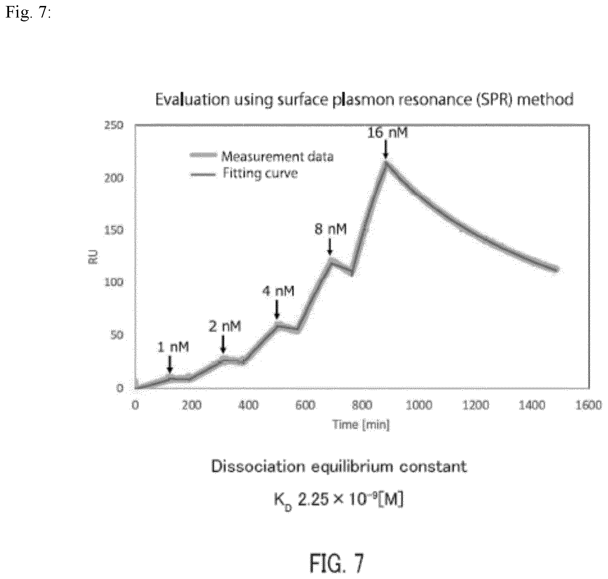

It was examined using a surface plasmon resonance method whether the Herceptin-Okt3 bispecific antibody obtained in Example 1(6) above could maintain a binding to Her2. Specifically, the Herceptin-Okt3 bispecific antibody was allowed to flow over HER2 immobilized on a sensor chip at a predetermined flow rate, and measurement was conducted using an intermolecular interaction analyzer. The Herceptin-Okt3 bispecific antibody purified in Example 1(6) above was diluted with 1×PBS-Tween20 to 1 nmol/l, 2 nmol/l, 4 nmol/l, 8 nmol/l, and 16 nmol/l. After the dilution, a surface plasmon resonance measuring apparatus (Biacore T200, manufactured by Cytiva) was used to measure the binding strength by allowing the diluted solution containing the Herceptin-Okt3 bispecific antibody to flow over HER2-hFc immobilized on a CM5 sensor chip (manufactured by GE Healthcare). FIG. 7 shows the results.

FIG. 7 shows a graph illustrating maintained binding of the bispecific antibody to HER2 measured through a surface plasmon resonance method. In FIG. 7, the horizontal axis indicates the time (minute), and the vertical axis indicates the response unit (RU). As shown in FIG. 7, when the Herceptin-Okt3 bispecific antibody obtained using the method of the present disclosure was added to HER2, the dissociation equilibrium constant, Kd value, was $2.25 \times 10^{-9}$ (M). It was found from these results that the Herceptin-Okt3 bispecific antibody obtained using the production method of the present invention maintained a binding to HER2.

As described above, the present invention has been described with reference to the embodiments, but the present invention is not limited to the above-described embodiments. Various modifications that can be understood by a person skilled in the art can be made in the configurations and details of the present invention without departing from the scope of the present invention.

The contents of the patents, patent applications, and references cited herein are incorporated herein by reference in their entireties as if specifically set forth herein.

<Supplementary Notes>

Some or all of the embodiments and the examples given above can be described as in the following supplementary notes, but are not limited thereto.

<Method for Producing Heterodimeric Protein>

(Supplementary Note 1)

A method for producing a heterodimeric protein, comprising a production step of producing a heterodimeric protein by reacting a dimeric protein having a reaction tag with a modification protein for modifying the dimeric protein,

the dimeric protein having a reaction tag comprising a first monomeric protein and a second monomeric protein,
the first monomeric protein comprising a first reaction tag and a first dimer formation domain capable of forming a dimer in this order,
the first reaction tag comprising a binding tag and a first C intein,
the second monomeric protein comprising a second reaction tag and a second dimer formation domain capable of forming a dimer together with the first dimer formation domain in this order,
the second reaction tag comprising a binding partner capable of binding to the binding tag, and a second C intein,
the modification protein comprising a first modification protein and a second modification protein,
the first modification protein comprising a first N intein capable of reacting with the first C intein, and a first addition component to be added to the first monomeric protein,
the second modification protein comprising a second N intein capable of reacting with the second C intein, and a second addition component to be added to the second monomeric protein,
the first addition component and the second addition component being different addition components, and
the first monomeric protein and the second monomeric protein forming a dimer, wherein, in the production step,
the first N intein of the first modification protein reacts with the first C intein of the first monomeric protein, and thus the first addition component of the first modification protein is linked to the first monomeric protein, and
the second N intein of the second modification protein reacts with the second C intein of the second monomeric protein, and thus the second addition component of the second modification protein is linked to the second monomeric protein.

(Supplementary Note 2)

The production method according to Supplementary Note 1, comprising a formation step of forming a dimer by reacting the first monomeric protein and the second monomeric protein.

(Supplementary Note 3)

The production method according to Supplementary Note 1 or 2, wherein the binding tag and the binding partner are a peptide tag and a peptide capable of spontaneously forming a covalent bond.

(Supplementary Note 4)

The production method according to any one of Supplementary Notes 1 to 3, wherein a binding between the binding tag and the binding partner is a covalent bond.

(Supplementary Note 5)

The production method according to any one of Supplementary Notes 1 to 4,

wherein the binding tag and the binding partner are a Streptococcus pyogenes surface protein (SpyCatcher) and a peptide tag (SpyTag) capable of binding to the SpyCatcher, respectively, or
the binding tag and the binding partner are the SpyTag and the SpyCatcher, respectively.

(Supplementary Note 6)

The production method according to any one of Supplementary Notes 1 to 5, wherein the dimeric protein comprises a portion or all of an Fc region of an immunoglobulin.

(Supplementary Note 7)

The production method according to Supplementary Note 6, wherein the immunoglobulin is IgG, IgA, IgE, IgD, or IgM.

(Supplementary Note 8)

The production method according to Supplementary Note 7, wherein the IgG is IgG1, IgG2, IgG2a, IgG2b, IgG3, or IgG4.

(Supplementary Note 9)

The production method according to any one of Supplementary Notes 6 to 8, wherein the Fc region of the immunoglobulin has no mutations.

(Supplementary Note 10)

The production method according to any one of Supplementary Notes 1 to 9,

wherein the first C intein and the first N intein are a C intein and an N intein of an integrated intein or a split-type intein, respectively, and/or
the second C intein and the second N intein are a C intein and an N intein of an integrated intein or a split-type intein, respectively.

(Supplementary Note 11)

The production method according to any one of Supplementary Notes 1 to 10, wherein the first addition component and/or the second addition component is a protein.

(Supplementary Note 12)

The production method according to Supplementary Note 11, wherein the protein is selected from the group consisting of an altered antibody, a heavy chain antibody (VHH), a receptor, a cytokine, a chemokine, a toxin, an enzyme, a fluorescent protein, and a collagen-binding domain.

(Supplementary Note 13)

The production method according to Supplementary Note 12, wherein the altered antibody is selected from the group consisting of Fab, a single-chain antibody (scFv), a tandem scFv, a heavy chain antibody (VHH), and a variable domain of the VHH.

(Supplementary Note 14)

The production method according to any one of Supplementary Notes 1 to 13,

wherein the dimeric protein comprises a portion or all of an Fc region of an immunoglobulin,
the first addition component and the second addition component comprise an altered antibody and/or a heavy chain antibody (VHH), and
the heterodimeric protein is a multispecific antibody.

(Supplementary Note 15)

The production method according to any one of Supplementary Notes 1 to 14, wherein, in the dimeric protein, the first monomeric protein and the second monomeric protein are linked together via a disulfide bond.

<Dimeric Protein>

(Supplementary Note 16)

A protein comprising a dimeric protein having a reaction tag,

wherein the dimeric protein having a reaction tag comprises a first monomeric protein and a second monomeric protein,

the first monomeric protein comprises a first reaction tag and a first dimer formation domain capable of forming a dimer,

the first reaction tag comprises a binding tag and a first C intein capable of reacting with a first N intein,

the second monomeric protein comprises a second reaction tag and a second dimer formation domain capable of forming a dimer together with the first dimer formation domain in this order,

the second reaction tag comprises a binding partner capable of binding to the binding tag, and a second C intein capable of reacting with a second N intein, and

the first monomeric protein and the second monomeric protein form a dimer.

(Supplementary Note 17)

The protein according to Supplementary Note 16, wherein the binding tag and the binding partner are a peptide tag and a peptide capable of spontaneously forming a covalent bond.

(Supplementary Note 18)

The protein according to Supplementary Note 16 or 17, wherein a binding between the binding tag and the binding partner is a covalent bond.

(Supplementary Note 19)

The protein according to any one of Supplementary Notes 16 to 18,

wherein the binding tag and the binding partner are a Streptococcus pyogenes surface protein (SpyCatcher) and a peptide tag (SpyTag) capable of binding to the SpyCatcher, respectively, or

the binding tag and the binding partner are the SpyTag and the SpyCatcher, respectively.

(Supplementary Note 20)

The protein according to any one of Supplementary Notes 16 to 19, wherein the dimeric protein comprises a portion or all of an Fc region of an immunoglobulin.

(Supplementary Note 21)

The protein according to Supplementary Note 20, wherein the immunoglobulin is IgG, IgA, IgE, IgD, or IgM.

(Supplementary Note 22)

The protein according to Supplementary Note 21, wherein the IgG is IgG1, IgG2, IgG2a, IgG2b, IgG3, or IgG4.

(Supplementary Note 23)

The protein according to any one of Supplementary Notes 20 to 22, wherein the Fc region of the immunoglobulin has no mutations.

(Supplementary Note 24)

The protein according to any one of Supplementary Notes 16 to 23,

wherein the first C intein and the first N intein are a C intein and an N intein of an integrated intein or a split-type intein, respectively, and/or

the second C intein and the second N intein are a C intein and an N intein of an integrated intein or a split-type intein, respectively.

<Monomeric Protein>

(Supplementary Note 25)

A protein comprising a first monomeric protein,

wherein the first monomeric protein comprises a first reaction tag and a first dimer formation domain capable of forming a dimer in this order,
the first reaction tag comprises a binding tag capable of binding to a binding partner and a first C intein capable of reacting with a first N intein,
the first monomeric protein is capable of forming a dimer together with a second monomeric protein,
the second monomeric protein comprises a second reaction tag and a second dimer formation domain capable of forming a dimer together with the first dimer formation domain, and
the second reaction tag comprises a binding partner capable of binding to the binding tag, and a second C intein capable of reacting with a second N intein.

(Supplementary Note 26)

A protein comprising a second monomeric protein,

wherein the second monomeric protein comprises a second reaction tag and a second dimer formation domain capable of forming a dimer in this order,
the second reaction tag comprises a binding partner capable of binding to a binding tag and a second C intein capable of reacting with a second N intein,
the second monomeric protein is capable of forming a dimer together with a first monomeric protein,
the first monomeric protein comprises a first reaction tag and a first dimer formation domain capable of forming a dimer together with the second dimer formation domain in this order, and
the first reaction tag comprises a binding tag capable of binding to the binding partner, and a first C intein capable of reacting with a first N intein.

(Supplementary Note 27)

The protein according to Supplementary Note 25 or 26, wherein the binding tag and the binding partner are a peptide tag and a peptide capable of spontaneously forming a covalent bond.

(Supplementary Note 28)

The protein according to any one of Supplementary Notes 25 to 27, wherein a binding between the binding tag and the binding partner is a covalent bond.

(Supplementary Note 29)

The protein according to any one of Supplementary Notes 25 to 28,

wherein the binding tag and the binding partner are a Streptococcus pyogenes surface protein (SpyCatcher) and a peptide tag (SpyTag) capable of binding to the SpyCatcher, respectively, or
the binding tag and the binding partner are the SpyTag and the SpyCatcher, respectively.

(Supplementary Note 30)

The protein according to any one of Supplementary Notes 25 to 29, wherein the dimeric protein comprises a portion or all of an Fc region of an immunoglobulin.

(Supplementary Note 31)

The protein according to Supplementary Note 30, wherein the immunoglobulin is IgG, IgA, IgE, IgD, or IgM.

(Supplementary Note 32)

The protein according to Supplementary Note 31, wherein the IgG is IgG1, IgG2, IgG2a, IgG2b, IgG3, or IgG4.

(Supplementary Note 33)

The protein according to any one of Supplementary Notes 30 to 32, wherein the portion or all of the Fc region of the immunoglobulin has no mutations.

(Supplementary Note 34)

The protein according to any one of Supplementary Notes 25 to 33,

wherein the first C intein and the first N intein are a C intein and an N intein of an integrated intein or a split-type intein, respectively, and/or
the second C intein and the second N intein are a C intein and an N intein of an integrated intein or a split-type intein, respectively.

<Nucleic Acid>

(Supplementary Note 35)

A nucleic acid encoding the protein according to any one of Supplementary Notes 25 and 27 to 34.

(Supplementary Note 36)

A nucleic acid encoding the protein according to any one of Supplementary Notes 26 to 34.

<Expression Vector>

(Supplementary Note 37)

An expression vector comprising the nucleic acid according to Supplementary Note 35 and/or the nucleic acid according to Supplementary Note 36.

(Supplementary Note 38)

The expression vector according to Supplementary Note 37, comprising a nucleic acid encoding a first modification protein and/or a nucleic acid encoding a second modification protein,

wherein the first modification protein comprises a first N intein capable of reacting with the first C intein, and a first addition component to be added to the first monomeric protein,
the second modification protein comprises a second N intein capable of reacting with the second C intein, and a second addition component to be added to the second monomeric protein, and
the first addition component and the second addition component are different addition components.

(Supplementary Note 39)

The expression vector according to Supplementary Note 38, wherein the first addition component and/or the second addition component is a protein.

(Supplementary Note 40)

The expression vector according to Supplementary Note 39, wherein the protein is selected from the group consisting of an altered antibody, a heavy chain antibody (VHH), a receptor, a cytokine, a chemokine, a toxin, a fluorescent protein, and a collagen-binding domain.

(Supplementary Note 41)

The expression vector according to Supplementary Note 40, wherein the altered antibody is selected from the group consisting of Fab, a single-chain antibody (scFv), a tandem scFv, a heavy chain antibody (VHH), and a variable domain of the VHH.

(Supplementary Note 42)

An expression vector set comprising an expression vector that comprises the nucleic acid according to Supplementary Note 35, and an expression vector that comprises the nucleic acid according to Supplementary Note 36.

(Supplementary Note 43)

The expression vector set according to Supplementary Note 42, comprising an expression vector that comprises a nucleic acid encoding a first modification protein, and/or an expression vector that comprises a nucleic acid encoding a second modification protein,

wherein the first modification protein comprises a first N intein capable of reacting with the first C intein, and a first addition component to be added to the first monomeric protein,
the second modification protein comprises a second N intein capable of reacting with the second C intein, and a second addition component to be added to the second monomeric protein, and
the first addition component and the second addition component are different addition components.

(Supplementary Note 44)

The expression vector set according to Supplementary Note 43, wherein the first addition component and/or the second addition component is a protein.

(Supplementary Note 45)

The expression vector set according to Supplementary Note 44, wherein the protein is selected from the group consisting of an altered antibody, a heavy chain antibody (VHH), a receptor, a cytokine, a chemokine, a toxin, a fluorescent protein, and a collagen-binding domain.

(Supplementary Note 46)

The expression vector set according to Supplementary Note 45, wherein the altered antibody is selected from the group consisting of Fab, a single-chain antibody (scFv), a tandem scFv, a heavy chain antibody (VHH), and a variable domain of the VHH.

<Transformant>

(Supplementary Note 47)

A transformant comprising the nucleic acid according to Supplementary Note 35, the nucleic acid according to Supplementary Note 36, the expression vector according to any one of Supplementary Notes 37 to 41, and/or the expression vector set according to any one of Supplementary Notes 42 to 45.

<Method for Producing Protein>

(Supplementary Note 48)

A method for producing a protein, comprising an expression step of expressing the nucleic acid according to Supplementary Note 35, the nucleic acid according to Supplementary Note 36, the expression vector according to any one of Supplementary Notes 37 to 41, and/or the expression vector set according to any one of Supplementary Notes 42 to 45.

(Supplementary Note 49)

The production method according to Supplementary Note 48,
wherein the expression step comprises:

a culturing step of culturing the transformant according to Supplementary Note 47; and
an isolation step of isolating a first monomeric protein and/or a second monomeric protein.

<Screening Method>

(Supplementary Note 50)

A method for screening a target-reactive heterodimeric protein, comprising:

a production step of producing a candidate heterodimeric protein by reacting a dimeric protein having a reaction tag with a modification protein for modifying the dimeric protein;
a detection step of detecting a reaction between the candidate heterodimeric protein and a target by bringing the candidate heterodimeric protein into contact with the target; and
a selection step of selecting the candidate heterodimeric protein used when the reaction is detected as a candidate of a substance that reacts with the target,
wherein the production step is conducted using the production method according to any one of Supplementary Notes 1 to 15.

(Supplementary Note 51)

The screening method according to Supplementary Note 50, wherein the reaction is a binding.

(Supplementary Note 52)

The screening method according to Supplementary Note 50 or 51, wherein the target is selected from the group consisting of a tumor antigen and a CD (cluster of differentiation) antigen.

(Supplementary Note 53)

The screening method according to any one of Supplementary Notes 50 to 52,

wherein the candidate hetero protein comprises a label, and
the label is detected in the detection step.

Industrial Applicability

As described above, with the present invention, heterodimeric proteins such as a bispecific antibody that are composed of only domains having natural amino acid sequences can also be produced. Also, with the present invention, a desired modification peptide can be applied to a dimeric protein. Accordingly, the present invention is very useful in the fields of pharmaceuticals, pharmaceutical production, and the like.

[Sequence Listing]

P22121WO.xml

**Claims**

1. A method for producing a heterodimeric protein, comprising a production step of producing a heterodimeric protein by reacting a dimeric protein having a reaction tag with a modification protein for modifying the dimeric protein,

the dimeric protein having a reaction tag comprising a first monomeric protein and a second monomeric protein,
the first monomeric protein comprising a first reaction tag and a first dimer formation domain capable of forming a dimer in this order,
the first reaction tag comprising a binding tag and a first C intein,
the second monomeric protein comprising a second reaction tag and a second dimer formation domain capable of forming a dimer together with the first dimer formation domain in this order,
the second reaction tag comprising a binding partner capable of binding to the binding tag, and a second C intein,
the modification protein comprising a first modification protein and a second modification protein,
the first modification protein comprising a first N intein capable of reacting with the first C intein, and a first addition component to be added to the first monomeric protein,

the second modification protein comprising a second N intein capable of reacting with the second C intein, and a second addition component to be added to the second monomeric protein,

the first addition component and the second addition component being different addition components, and

the first monomeric protein and the second monomeric protein forming a dimer,

wherein, in the production step,

the first N intein of the first modification protein reacts with the first C intein of the first monomeric protein, and thus the first addition component of the first modification protein is linked to the first monomeric protein, and

the second N intein of the second modification protein reacts with the second C intein of the second monomeric protein, and thus the second addition component of the second modification protein is linked to the second monomeric protein.

2. The production method according to claim 1, comprising a formation step of forming a dimer by reacting the first monomeric protein and the second monomeric protein.

3. The production method according to claim 1 or 2, wherein the binding tag and the binding partner are a peptide tag and a peptide capable of spontaneously forming a covalent bond.

4. The production method according to claim 1 or 2, wherein a binding between the binding tag and the binding partner is a covalent bond.

5. The production method according to claim 1 or 2,

wherein the binding tag and the binding partner are a Streptococcus pyogenes surface protein (SpyCatcher) and a peptide tag (SpyTag) capable of binding to the SpyCatcher, respectively, or

the binding tag and the binding partner are the SpyTag and the SpyCatcher, respectively.

6. The production method according to claim 1 or 2, wherein the dimeric protein comprises a portion or all of an Fc region of an immunoglobulin.

7. The production method according to claim 6, wherein the immunoglobulin is IgG, IgA, IgE, IgD, or IgM.

8. The production method according to claim 7, wherein the IgG is IgG1, IgG2, IgG2a, IgG2b, IgG3, or IgG4.

9. The production method according to claim 6 or 7, wherein the Fc region of the immunoglobulin has no mutations.

10. The production method according to claim 1 or 2,

wherein the first C intein and the first N intein are a C intein and an N intein of an integrated intein or a split-type intein, respectively, and/or

the second C intein and the second N intein are a C intein and an N intein of an integrated intein or a split-type intein, respectively.

11. The production method according to claim 1 or 2, wherein the first addition component and/or the second addition component is a protein.

12. The production method according to claim 11, wherein the protein is selected from the group consisting of an altered antibody, a heavy chain antibody (VHH), a receptor, a cytokine, a chemokine, a toxin, an enzyme, a fluorescent protein, and a collagen-binding domain.

13. The production method according to claim 12, wherein the altered antibody is selected from the group consisting of Fab, a single-chain antibody (scFv), a tandem scFv, a heavy chain antibody (VHH), and a variable domain of the VHH.

14. The production method according to claim 1 or 2,

wherein the dimeric protein comprises a portion or all of an Fc region of an immunoglobulin,

the first addition component and the second addition component comprise an altered antibody and/or a heavy chain antibody (VHH), and

the heterodimeric protein is a multispecific antibody.

15. The production method according to claim 1 or 2, wherein, in the dimeric protein, the first monomeric protein and the second monomeric protein are linked together via a disulfide bond.

16. A protein comprising a dimeric protein having a reaction tag,

wherein the dimeric protein having a reaction tag comprises a first monomeric protein and a second monomeric protein,
the first monomeric protein comprises a first reaction tag and a first dimer formation domain capable of forming a dimer,
the first reaction tag comprises a binding tag and a first C intein capable of reacting with a first N intein,
the second monomeric protein comprises a second reaction tag and a second dimer formation domain capable of forming a dimer together with the first dimer formation domain in this order,
the second reaction tag comprises a binding partner capable of binding to the binding tag, and a second C intein capable of reacting with a second N intein, and
the first monomeric protein and the second monomeric protein form a dimer.

17. A protein comprising a first monomeric protein,

wherein the first monomeric protein comprises a first reaction tag and a first dimer formation domain capable of forming a dimer in this order,
the first reaction tag comprises a binding tag capable of binding to a binding partner and a first C intein capable of reacting with a first N intein,
the first monomeric protein is capable of forming a dimer together with a second monomeric protein,
the second monomeric protein comprises a second reaction tag and a second dimer formation domain capable of forming a dimer together with the first dimer formation domain, and
the second reaction tag comprises a binding partner capable of binding to the binding tag, and a second C intein capable of reacting with a second N intein.

18. A protein comprising a second monomeric protein,

wherein the second monomeric protein comprises a second reaction tag and a second dimer formation domain capable of forming a dimer in this order,
the second reaction tag comprises a binding partner capable of binding to a binding tag and a second C intein capable of reacting with a second N intein,
the second monomeric protein is capable of forming a dimer together with a first monomeric protein,
the first monomeric protein comprises a first reaction tag and a first dimer formation domain capable of forming a dimer together with the second dimer formation domain in this order, and
the first reaction tag comprises a binding tag capable of binding to the binding partner, and a first C intein capable of reacting with a first N intein.

19. A nucleic acid encoding the protein according to claim 16.

20. A nucleic acid encoding the protein according to claim 17.

21. A nucleic acid encoding the protein according to claim 18.

22. An expression vector comprising a nucleic acid of the nucleic acid according to claim 19.

23. An expression vector set comprising an expression vector having the nucleic acid according to claim 20 and the nucleic acid according to claim 21.

24. A method for producing a protein, comprising an expression step of expressing the nucleic acid according to any one of claims 19 to 21, the expression vector according to claim 22, and/or an expression vector set that comprises an expression vector having the nucleic acid according to claim 20 and the nucleic acid according to claim 21.

25. A method for screening a target-reactive heterodimeric protein, comprising:

a production step of producing a candidate heterodimeric protein by reacting a dimeric protein having a reaction tag with a modification protein for modifying the dimeric protein;

a detection step of detecting a reaction between the candidate heterodimeric protein and a target by bringing the candidate heterodimeric protein into contact with the target; and

a selection step of selecting the candidate heterodimeric protein used when the reaction is detected as a candidate of a substance that reacts with the target,

wherein the production step is conducted using the production method according to claim 1 or 2.

26. The screening method according to claim 25, wherein the reaction is a binding.

27. The screening method according to claim 25 or 26, wherein the target is selected from the group consisting of a tumor antigen and a CD (cluster of differentiation) antigen.

28. The screening method according to claim 25 or 27,

wherein the candidate hetero protein comprises a label, and
the label is detected in the detection step.

Fig. 1:

FIG. 1A

FIG. 1B

FIG. 1C

FIG. 1D

Fig. 2:

FIG. 2

Fig. 3:

(A) KanCapA (Fc region) column used

Marker | Reaction 24 hours | Flow-through | Washed | Eluted

kDa
66.4
44.3
29.0
20.1

Contaminating proteins

12.5% acrylamide gel used

FIG. 3A

(B) KanCapL (Fab region) column used

Eluted Reduced

kDa

50.1 kDa
49.7 kDa
23.3 kDa
23.4 kDa

66.4
44.3
29.0

10% acrylamide gel used

FIG. 3B

Fig. 4:

FIG. 4

Fig. 5:

FIG. 5

Fig. 6:

FIG. 6

Fig. 7:

Evaluation using surface plasmon resonance (SPR) method

Dissociation equilibrium constant

$K_D \ 2.25 \times 10^{-9}[M]$

FIG. 7

## INTERNATIONAL SEARCH REPORT

International application No.

**PCT/JP2023/019733**

### A. CLASSIFICATION OF SUBJECT MATTER

*C12N 15/62*(2006.01)i; *C07K 14/315*(2006.01)i; *C07K 14/52*(2006.01)i; *C07K 14/705*(2006.01)i; *C07K 16/00*(2006.01)i;
*C07K 19/00*(2006.01)i; *C12N 15/63*(2006.01)i; *C12P 21/00*(2006.01)i; *G01N 33/50*(2006.01)i
FI: C12N15/62 Z; C07K14/315 ZNA; C07K14/52; C07K14/705; C07K16/00; C07K19/00; C12N15/63 Z; C12P21/00 C;
G01N33/50 Z

According to International Patent Classification (IPC) or to both national classification and IPC

### B. FIELDS SEARCHED

Minimum documentation searched (classification system followed by classification symbols)

C12N15/62; C07K14/315; C07K14/52; C07K14/705; C07K16/00; C07K19/00; C12N15/63; C12P21/00; G01N33/50

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| A | WU, W. H. et al. Higher Order Protein Catenation Leads to an Artificial Antibody with Enhanced Affinity and In Vivo Stability. J. Am. Chem. Soc. 2021, vol. 143, pp. 18029-18040 abstract, scheme 3 | 1-15, 25-28 |
| X | | 16-24 |
| A | CN 111560391 A (PEKING UNIVERSITY) 21 August 2020 (2020-08-21) example 1, fig. 1-2 | 1-15, 25-28 |
| X | | 16-24 |
| A | JP 2019-506163 A (THE TRUSTEES OF PRINCETON UNIVERSITY) 07 March 2019 (2019-03-07) entire text, all drawings | 1-28 |
| A | US 2019/0062434 A1 (SHANGHAI JIAO TONG UNIVERSITY) 28 February 2019 (2019-02-28) whole document | 1-28 |

☑ Further documents are listed in the continuation of Box C. ☑ See patent family annex.

| | | |
|---|---|---|
| * | Special categories of cited documents: | |
| "A" | document defining the general state of the art which is not considered to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **13 July 2023** | **25 July 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

International application No.

**PCT/JP2023/019733**

| C. | DOCUMENTS CONSIDERED TO BE RELEVANT | |
|---|---|---|
| Category* | Citation of document, with indication, where appropriate, of the relevant passages | Relevant to claim No. |
| A | US 2020/0277403 A1 (THE TRUSTEES OF THE UNIVERSITY OF PENNSYLVANIA) 03 September 2020 (2020-09-03) <br> whole document | 1-28 |

Form PCT/ISA/210 (second sheet) (January 2015)

**INTERNATIONAL SEARCH REPORT**

International application No.

**PCT/JP2023/019733**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    "Annex C/ST.25 text file format" should be read as 'ST.26 format"

Form PCT/ISA/210 (continuation of first sheet) (January 2015)

**INTERNATIONAL SEARCH REPORT**
**Information on patent family members**

| International application No. |
| --- |
| **PCT/JP2023/019733** |

| Patent document cited in search report | | | Publication date (day/month/year) | Patent family member(s) | | | Publication date (day/month/year) |
| --- | --- | --- | --- | --- | --- | --- | --- |
| CN | 111560391 | A | 21 August 2020 | WO | 2021/233330 | A1 | |
| JP | 2019-506163 | A | 07 March 2019 | US | 2020/0055900 | A1 | |
| | | | | whole document | | | |
| | | | | WO | 2017/132580 | A2 | |
| | | | | EP | 3408292 | A2 | |
| US | 2019/0062434 | A1 | 28 February 2019 | WO | 2017/143838 | A1 | |
| | | | | EP | 3418305 | A1 | |
| US | 2020/0277403 | A1 | 03 September 2020 | WO | 2018/053180 | A2 | |
| | | | | EP | 3512564 | A2 | |

Form PCT/ISA/210 (patent family annex) (January 2015)

## REFERENCES CITED IN THE DESCRIPTION

**Patent documents cited in the description**

- WO 2013065708 A

**Non-patent literature cited in the description**

- **G. WOZNIAK-KNOPP et al.** Introducing antigen-binding sites in structural loops of immunoglobulin constant domains: Fc fragments with engineered HER2/neu-binding sites and antibody properties. *Protein Engineering, Design and Selection*, April 2010, vol. 23 (4), 289-297
- **SOPP, J.M. et al.** On-target IgG hexamerisation driven by a C-terminal IgM tail-piece fusion variant confers augmented complement activation. *Commun. Biol.*, 2021, vol. 4, 1031
- **DE JONG RN et al.** A Novel Platform for the Potentiation of Therapeutic Antibodies Based on Antigen-Dependent Formation of IgG Hexamers at the Cell Surface. *PLoS. Biol.*, 2016, vol. 14 (1), e1002344
- **CHRISTOPH A. DIEBOLDER et al.** Complement Is Activated by IgG Hexamers Assembled at the Cell Surface. *Science*, vol. 343 (6176), 1260-1263
- **CHRISTOPH SPIESS et al.** Alternative molecular formats and therapeutic applications for bispecific antibodies. *Molecular Immunology*, 2015, vol. 67 (2), 95-106
- **ANTHONY H. KEEBLE et al.** Evolving Accelerated Amidation by SpyTag/SpyCatcher to Analyze Membrane Dynamics. *Angew. Chem. Int. Ed.*, 2017, vol. 56, 16521-16525
- **ANTHONY H. KEEBLE et al.** Approaching infinite affinity through engineering of Peptide-protein interaction. *PNAS*, 2019, vol. 116 (52), 26523-26533
- **VEGGIANI G ; NAKAMURA T ; BRENNER MD ; GAYET RV ; YAN J ; ROBINSON CV ; HOWARTH M**. Programmable polyproteams built using twin peptide superglues. *Proc Natl Acad Sci U S A.*, 02 February 2016, vol. 113 (5), 1202-7
- **CIRAGAN A. et al.** Salt-inducible Protein Splicing in cis and trans by Inteins from Extremely Halophilic Archaea as a Novel Protein-Engineering Tool. *J Mol Biol.*, 06 October 2016, vol. 428 (23), 4573-4588
- **ZEIDLER, M. et al.** Temperature-sensitive control of protein activity by conditionally splicing inteins. *Nat Biotechnol*, 2004, vol. 22, 871-876, https://doi.org/10.1038/nbt979